# EUROPEAN PATENT APPLICATION

(11) **EP 2 172 156 A1**
(43) Date of publication of application: **07.04.2010**
(21) Application number: 09252294.5
(22) Date of filing: 29.09.2009
(51) Int. Cl.: A61B 17/00

(54) **Methods and devices for performing gastrectomies and gastroplasties**

(30) Priority: 30.09.2008 US 242333
(71) Applicant: Ethicon Endo-Surgery, Inc., Cincinnati, Ohio 45242-2839 (US)
(72) Inventor: Hess, Christopher J., Cincinnati, Ohio 45206 (US); Murray, Michael A., Bellevue, Kentucky 41073 (US); Gill, Robert P., Mason, Ohio 45040 (US); Voegele, James Walden, Cincinnati, Ohio 45249 (US); Weisenburgh, II, William Bruce, Maineville, Ohio 45039 (US); Powell, Darrel M., Cincinnati, Ohio 45249 (US); Myers, Stephen Ray, Columbus, Ohio 43235 (US)
(74) Representative: Tunstall, Christopher Stephen

(57) **Abstract**

Methods and devices are provided for performing gastrectomies and gastroplasties. In one embodiment, a method includes gaining access to a stomach of a patient through an opening formed in the patient's abdominal wall and an opening formed in the patient's vaginal wall. Tissue attached to the stomach can be tensioned using a surgical instrument inserted through one of the abdominal and vaginal openings and can be separated from the stomach to free the stomach fundus using a dissecting surgical instrument inserted through another opening, e.g., through one of the abdominal and vaginal openings. The fundus can be at least partially transected using a surgical stapler inserted through one of the abdominal and vaginal openings, thereby forming a stomach "sleeve." In another embodiment, the method is modified to form another opening in the patient's abdominal wall instead of forming an opening in the vaginal wall.

## Description

### FIELD OF THE INVENTION

The present invention relates to gastrectomies and gastroplasties and methods and devices for performing gastrectomies and gastroplasties.

### BACKGROUND OF THE INVENTION

Obesity is becoming a growing concern, particularly in the United States, as the number of obese people continues to increase and more is learned about the negative health effects of obesity. Morbid obesity, in which a person is 100 pounds or more over ideal body weight, in particular poses significant risks for severe health problems. Accordingly, a great deal of attention is being focused on treating obese patients. Surgical procedures to treat morbid obesity have included gastric bypasses (stomach stapling), adjustable gastric banding, and vertical banded gastroplasty and sleeve gastrectomies (removal of all or a portion of the stomach). Such surgical procedures have increasingly been performed laparoscopically. Reduced postoperative recovery time, markedly decreased post-operative pain and wound infection, and improved cosmetic outcome are well established benefits of laparoscopic surgery, derived mainly from the ability of laparoscopic surgeons to perform an operation utilizing smaller incisions of the body cavity wall. However, multiple abdominal incisions are often required in such obesity treatment procedures, thereby increasing chances for undesirable post-operative consequences such as cosmetic scarring.

Sleeve gastrectomies and gastroplasties have become increasingly favored by surgeons and patients for treating obesity, as well as for treating stomach diseases such as cancer where a portion of the stomach is removed, because sleeve gastrectomies and gastroplasties do not leave any foreign material in a patient and do not require a complicated intestinal bypass. Instead, the stomach's volume is reduced through partial removal or division of the stomach, thereby leaving a stomach "sleeve" between the esophagus and intestine. A laparoscopic sleeve gastrectomy or gastroplasty procedure generally involves insufflation of the abdominal cavity with carbon dioxide gas to a pressure of around 15 millimeters of mercury (mm Hg). The abdominal wall is pierced and a 5-10 mm in diameter straight tubular cannula or trocar is then inserted into the abdominal cavity. A laparoscopic telescope connected to an operating room monitor is used to visualize the operative field and is placed through one of the trocar(s). Laparoscopic instruments are placed through two or more additional trocars for manipulation by the surgeon and surgical assistant(s). Thus, such laparoscopic procedures can require multiple instruments to be introduced into a patient through multiple, potentially scarring incisions and/or can result in interference between instruments near each other. The placement of two or more standard cannulas and laparoscopic instruments in the abdomen next to each other and/or placement of two or more instruments into the abdomen through the same incision creates a so-called "chopstick" effect, which describes interference between the surgeon's hands, between the surgeon's hands and the instruments, and between the instruments. This interference greatly reduces the surgeon's ability to perform a described procedure.

Accordingly, there remains a need for methods and devices for performing gastrectomies and gastroplasties that minimize patient recovery time, improve cosmetic outcome, and reduce the "chopstick" effect.

### SUMMARY OF THE INVENTION

The present invention generally provides methods and devices for performing gastrectomies and gastroplasties. In one embodiment, a surgical method is provided that includes forming a first access hole in an abdominal wall of a patient and forming a second access hole through a digestive tract of the patient and into an abdominal cavity of the patient. The method can also include tensioning a tissue attached to a stomach of the patient using a first surgical instrument inserted through one of the first and second access holes, detaching the tissue from the stomach using a second surgical instrument inserted through one of the first and second access holes, and transecting a portion of the stomach using a surgical stapler inserted through one of the first and second access holes to form a stomach sleeve. The method can also include removing the transected portion of the stomach through one of the first and second access holes. The various steps can be performed under visualization, for example by advancing a scoping device with a viewing element located thereon into the patient through one of the first and second access holes.

Various instruments can be used to facilitate formation of a stomach sleeve. For example, the method can include transorally introducing a sizing device into the stomach and using the sizing device to size the portion of the stomach to be transected. As still another example, forming the first access hole can include positioning a housing having a plurality of sealing ports in the abdominal wall.

In other embodiments, transecting a portion of the stomach can include separating a fundus of the stomach from an area of the stomach substantially near an esophagus of the patient, wherein the fundus retains fluid communication with a pyloric valve of the patient. For yet another example, the second access hole can be formed in the stomach sleeve, and, in some embodiments, the method can include retracting a liver of the patient using a device inserted through one of the first and second access holes.

In another aspect, methods and devices for performing gastrectomies and gastroplasties are provided and include forming an access hole in a vaginal wall of a patient. In one embodiment, a surgical method is provided that includes forming a first access hole in an abdominal wall of a patient by positioning a housing having a plurality of sealing ports in the abdominal wall, forming a second access hole in a vaginal wall of the patient, tensioning a tissue attached to a stomach of the patient using a first surgical instrument inserted through the second access hole, detaching the tissue from the stomach using a second surgical instrument inserted through one of the sealing ports in the housing, and transecting a portion of the stomach using a third surgical instrument inserted through one of the sealing ports in the housing. The method can have any number of variations. For example, the method can include retracting a liver of the patient using a device inserted through one of the sealing ports in the housing. As another example, the method can include removing the transected portion of the stomach from the patient through one of the first and second access holes. As yet another example, the first surgical instrument can include a first grasper, and the method can include tensioning the tissue using a second grasper inserted through the second access hole. As still another example, the method can include transorally introducing a sizing device into the stomach and using the sizing device to size the portion of the stomach to be transected. As another example, the method can include advancing a scoping device with a viewing element located thereon into the patient through the second access hole. Advancing the scoping device can include bending the scoping device in the stomach in at least two directions relative to a longitudinal axis of the scoping device to visualize the stomach in a direction toward a fundus of the stomach.

In another embodiment, a surgical method is provided that includes forming first and second access holes in an abdominal wall of a patient, forming a third access hole in a vaginal wall of the patient, tensioning a tissue attached to the stomach using a surgical instrument inserted through the second access hole, visualizing the stomach using a scoping device with a viewing element located thereon inserted into the patient through the third access hole, and transecting a portion of a stomach of the patient less than an entire length of the stomach using a surgical stapler inserted through the first access hole. The first access hole can have a diameter greater than a diameter of the second access hole. The method can have any number of variations. For example, the method can include retracting a liver of the patient using a device inserted into the patient through the first access hole. For another example, transecting a portion of the stomach can include separating a fundus of the stomach from an area of the stomach substantially near an esophagus of the patient, wherein the fundus retains fluid communication with a pyloric valve of the patient. For yet another example, visualizing the stomach using a scoping device can include bending the scoping device in the stomach in at least two directions relative to a longitudinal axis of the scoping device to visualize the stomach in a direction toward a fundus of the stomach. Bending the scoping device in the stomach can include bending the scoping device in a first direction at a first location along the longitudinal axis and bending an overtube disposed over the scoping device in a second direction at a second location along the longitudinal axis. For still another example, the method can include transorally introducing a sizing device into the stomach and using the sizing device to size the portion of the stomach to be transected. As another example, the method can include mounting the scoping device to a support external to the patient. As yet another example, the method can include inserting a grasper through the third access hole to tension a tissue surrounding the stomach. As still another example, forming the third access hole can include piercing the vaginal wall using an optically clear tapered tip of a surgical instrument. As another example, the method can include sedating the patient using a conscious sedation system. As yet another example, the method can include delivering a drug-eluting device into the stomach.

In another embodiment, a surgical method is provided that includes forming first and second access holes in an abdominal wall of a patient, forming a third access hole in a vaginal wall of the patient, tensioning a tissue attached to a stomach of the patient using a first surgical instrument inserted through the second access hole, detaching the tissue from the stomach using a second surgical instrument inserted through the first access hole, and transecting a portion of the stomach using a third surgical instrument inserted through the first access hole. The first access hole can have a diameter greater than a diameter of the second access hole. The method can have any number of variations. For example, detaching the tissue can occur when the tissue is being tensioned using the first surgical instrument. As another example, the first surgical instrument can include at least one grasper, and the second surgical instrument can include a harmonic scalpel. As yet another example, the method can include transorally introducing a sizing device into the stomach and using the sizing device to size the portion of the stomach to be transected. As still another example, forming the third access hole can include piercing the vaginal wall using an optically clear tapered tip of a surgical instrument. As another example, the method can include sedating the patient using a conscious sedation system and/or delivering a drug-eluting device into the stomach. As yet another example, the method can include advancing a scoping device with a viewing element located thereon into the patient through the third access hole. Advancing a scoping device can include bending the scoping device in the stomach in at least two directions relative to a longitudinal axis of the scoping device to visualize the stomach in a direction toward a fundus of the stomach. Bending the scoping device in the stomach can include bending the scoping device in a first direction at a first location along the longitudinal axis and bending an overtube disposed over the scoping device in a second direction at a second location along the longitudinal axis. The method can further include mounting the scoping device to a support external to the patient and/or inserting a grasper through the third access hole to tension a tissue surrounding the stomach.

In another embodiment, a surgical method is provided that includes forming a first access hole in an abdominal wall of a patient, forming a second access hole in a vaginal wall of the patient, tensioning a tissue attached to a stomach of the patient using a first surgical instrument inserted through the second access hole, detaching the tissue from the stomach using a second surgical instrument inserted through the first access hole, and transecting a portion of the stomach using a surgical stapler inserted through the second access hole. The method can have any number of variations. For example, tensioning a tissue can include using at least two graspers inserted through the second access hole to tension the tissue. For another example, the method can include manipulating stomach tissue to be stapled by the surgical stapler using at least one grasper inserted through the first access hole. For yet another example, forming a first access hole can include positioning a housing having a plurality of sealing ports in the abdominal wall. As another example, the method can include advancing a scoping device with a viewing element located thereon into the patient through the first access hole. As still another example, the method can include removing the transected portion of the stomach from the patient through one of the first and second access holes. For another example, the method can include transorally introducing a sizing device into the stomach and using the sizing device to size the portion of the stomach to be transected.

### BRIEF DESCRIPTION OF THE DRAWINGS

The invention will be more fully understood from the following detailed description taken in conjunction with the accompanying drawings, in which:

FIG. 1 is a perspective partially transparent view of one embodiment of a patient having an access hole formed in an abdominal wall of the patient;

FIG. 2 is a perspective partially transparent view of one embodiment of a patient having an access hole formed in a vaginal wall of the patient;

FIG. 3 is a side view of one embodiment of a rigid obturator;

FIG. 4 is a side view of one embodiment of a flexible obturator;

FIG. 5 is a side view of one embodiment of an articulating trocar;

FIG. 6 is a perspective partially transparent view of one embodiment of a flexible standalone sleeve;

FIG. 7 is a perspective view of one embodiment of a rigid standalone sleeve having surgical devices extending therethrough;

FIG. 8 is a cross-sectional view of the sleeve of FIG. 7;

FIG. 9 is a perspective view of another embodiment of a rigid standalone sleeve having surgical devices extending therethrough;

FIG. 10 is a perspective partially exploded view of another embodiment of a standalone sleeve;

FIG. 11 is a perspective view of one embodiment of a distal end of an endoscope flexed in two distal regions;

FIG. 12 is a perspective view of one embodiment of a support for mounting an endoscope to an examination table;

FIG. 13 is a perspective partially transparent view of an alternate embodiment of a device holder for the support of FIG. 12;

FIG. 14 is a side view of another alternate embodiment of a device holder for the support of FIG. 12;

FIG. 15 is a perspective view of another alternate embodiment of a device holder for the support of FIG. 12;

FIG. 16 is a side view of an alternate embodiment of a mount for the support of FIG. 12;

FIG. 17 is a side partially transparent view of an embodiment of multi-arm adapter for the support of FIG. 12;

FIG. 18 is a top view of another embodiment of multi-arm adapter for the support of FIG. 12;

FIG. 19 is a perspective partially transparent view of one embodiment of a liver retracting device retracting a liver of a patient;

FIG. 20 is a perspective partially cross-sectional view of the liver retracting device of FIG. 19;

FIG. 21 is a perspective partially transparent view of a liver of a patient being retracted by a retractor inserted through a multiple port access device;

FIG. 22 is a perspective view of one embodiment of the multiple port access device of FIG. 21;

FIG. 23 is a perspective partially transparent view of one embodiment of a tacker device shown retracting a liver of a patient;

FIG. 24 is a perspective partially transparent view of one embodiment of a sizer advanced into a stomach of a patient;

FIG. 25 is a side cross-sectional view of the patient and sizer of FIG. 24;

FIG. 26 is a perspective partially transparent view of one embodiment of a dissecting device dissecting tissue from a stomach of a patient;

FIG. 27 is a perspective partially transparent view of one embodiment of a dissecting device dissecting tissue from a stomach of a patient using a multiple port access device;

FIG. 28 is a perspective partially transparent view of one embodiment of a vaginally inserted dissecting device dissecting tissue from a stomach of a patient;

FIG. 29 is a perspective partially transparent view of one embodiment of a dissecting device dissecting tissue from a stomach of a patient with a grasper tensioning the tissue;

FIG. 30 is a perspective partially transparent view of one embodiment of a dissecting device dissecting tissue from a stomach of a patient with a grasper tensioning the tissue and advanced through an opening in a digestive tract of the patient;

FIG. 31 is a perspective partially transparent view of one embodiment of a patient having an access hole formed in a vaginal wall of the patient, a first abdominal port formed at an umbilicus of the patient, and a second abdominal port formed in an abdominal wall of the patient;

FIG. 32 is a perspective partially transparent view of one embodiment of a transecting device transecting a stomach of a patient and inserted into the patient through an access hole formed in a vaginal wall of the patient;

FIG. 33 is a perspective partially transparent view of one embodiment of a transecting device transecting a stomach of a patient and inserted into the patient through a multiple port access device disposed in an abdomen of the patient; and

FIG. 34 is a perspective partially transparent view of one embodiment of a patient having a first abdominal port formed at an umbilicus of the patient and second and third abdominal ports formed in an abdominal wall of the patient.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Various exemplary methods and devices are provided for performing gastrectomies and gastroplasties. In certain embodiments, a method of performing a gastrectomy or a gastroplasty can include gaining access to a stomach of a patient through one or more openings formed in one or more of the patient's digestive tract, abdominal wall, and vaginal wall. Various instruments can be inserted through the various openings to perform certain steps, such as tensioning and cutting tissue, sizing and transecting the stomach, viewing the surgical site, etc.

A patient can be prepared for a gastrectomy or gastroplasty surgical procedure in any way, as will be appreciated by a person skilled in the art. For example, the patient can be fully sedated or consciously sedated for the procedure. Non-limiting embodiments of a conscious sedation system can be found in U.S. Patent Publication No. 2006/0042636 filed on June 21, 2005 and entitled "Oral Nasal Cannula," U.S. Patent No. 6,807,965 issued October 26, 2004 and entitled "Apparatus And Method For Providing A Conscious Patient Relief From Pain And Anxiety Associated With Medical Or Surgical Procedures," U.S. Patent No. 7,201,734 issued April 10, 2007 and entitled "Apparatus For Drug Delivery In Association With Medical Or Surgical Procedures," U.S. Patent No. 7,247,154 issued July 24, 2007 and entitled "Method For Drug Delivery In Association With Medical Or Surgical Procedures," which are hereby incorporated by reference in their entireties.

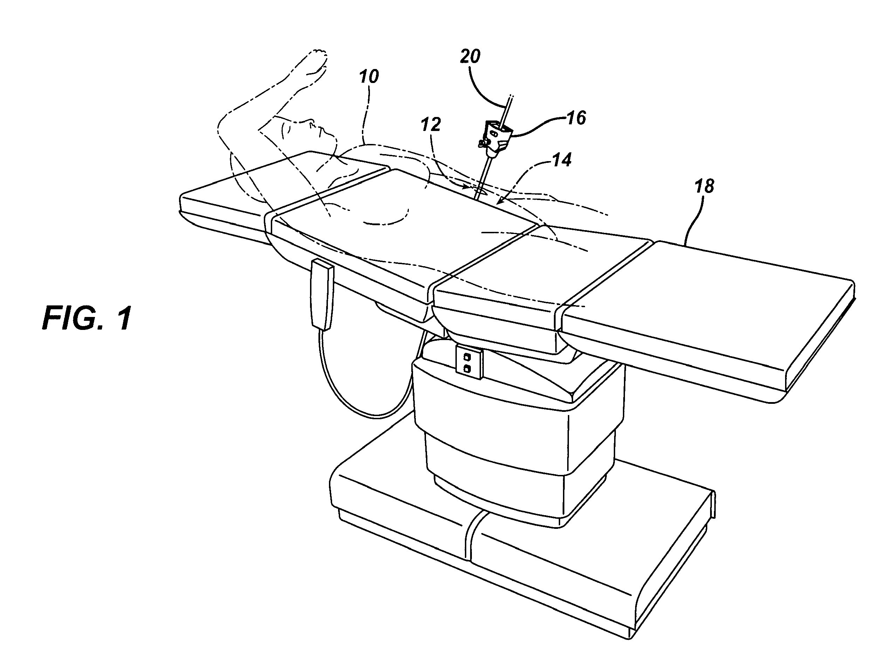

In one exemplary embodiment of a gastrectomy procedure illustrated in FIG. 1, an abdominal opening or access hole 12 can be formed in an abdominal wall 14 of a patient 10. During the gastrectomy, the patient 10 is preferably positioned as shown in a reclined, substantially horizontal lithotomy position on an examination table 18 to provide clear access to the patient's abdominal region. FIG. 1 and other figures discussed herein are simplified for ease of presentation and do not always illustrate the patient 10 and/or devices present at a given moment in a surgical procedure, such as devices shown in one or more previously described figures and any additional necessary equipment, e.g., patient monitoring equipment, safety devices, video monitors, etc. The gastrectomy is described as performed by a surgeon, but as will be appreciated by a person skilled in the art, one or more medical professionals, e.g., surgeons, surgical assistants, nurses, etc., can perform any one or more portions of the procedure. Furthermore, the surgical procedure is referred to as a gastrectomy, but a person skilled in the art will appreciate that the methods and devices can be similarly applicable to a gastroplasty, such as a Magenstrasse and Mill procedure in which only a portion of the stomach is transected.

As shown in FIG. 1, the abdominal opening or access hole 12 can be formed in the patient's abdominal wall 14. Smaller and fewer body cavity incisions can generally improve a patient's recovery time and reduce pain, so it can be advantageous to perform an operation utilizing only a single abdominal incision in the navel. The umbilicus is the thinnest and least vascularized, and a well-hidden area of the abdominal wall 14. An umbilical incision can be easily enlarged, e.g., in order to eviscerate a larger specimen, without significantly compromising cosmesis and without increasing the chances of wound complications. The abdominal access hole 12 can be in the form of a substantially circular otomy, or it can be a percutaneous incision as shown. A person skilled in the art will appreciate that the term "otomy" as used herein is intended to encompass a larger opening or access hole having an outer diameter of about 25.4 mm (about 1 inch) and that the term "percutaneous opening" or "percutaneous access hole" as used herein is intended to encompass a relatively small opening or access hole in a patient that preferably has a diameter in a range of about 3 to 5 mm.

The abdominal access hole 12 can be formed in any way, as will be appreciated by a person skilled in the art. As illustrated, the abdominal access hole 12 is formed using a trocar 16. The trocar 16 can include any cannula configured to incise tissue and having a cannulated interior through which a surgical instrument can be passed into a patient through the incised tissue. The trocar 16 can include also an optical tip configured to provide visualization of the abdominal wall 14 as the trocar 16 is passed therethrough, for example using a scoping device with a viewing element located thereon, e.g., a laparoscope 20, that is inserted into the trocar 16. The laparoscope 20 can be inserted into the trocar 16 at any time, including during penetration through the tissue or after the trocar 16 penetrates the abdominal wall 14. A person skilled in the art will also appreciate that a scoping device used in the gastrectomy can include any surgical device having a viewing element, e.g., a lens, located thereon. Non-limiting examples of a scoping device include an endoscope, a laparoscope, a gastroscope, and a colonoscope. The laparoscope 20, as well as the other devices discussed herein, can be made from any combination of rigid and/or flexible materials, but in an exemplary embodiment the materials are biocompatible. A person skilled in the art will appreciate that the term "flexible" as used herein is intended to encompass a variety of configurations. Generally, a "flexible" member has some degree of elasticity, e.g., is capable of bending without breaking. In an exemplary embodiment, a flexible device or at least portions thereof are composed of at least one biocompatible and flexible material, e.g., plastic, titanium, stainless steel, etc. Various portions of a flexible device can also be formed from a shape memory material, such as Nitinol.

Once access to the abdominal cavity is obtained, the surgeon can insufflate the patient's abdominal cavity through the abdominal access hole 12, as will be appreciated by a person skilled in the art, to expand the abdominal cavity and provide a larger, more easily navigable surgical workspace. For example, the surgeon can insufflate the abdominal cavity by passing a fluid under pressure, e.g., nontoxic carbon dioxide gas, through the trocar 16. The fluid can have a pressure in the range of about 10 to 15 mm Hg, or any other pressure, as will be appreciated by a person skilled in the art. The trocar 16 can include one more seals that prevent the insufflation fluid from escaping the abdominal cavity through the trocar 16. A non-limiting example of a sealing trocar that does not use seals is the SurgiQuest AirSeal^{™} available from SurgiQuest, Inc. of Orange, Connecticut.

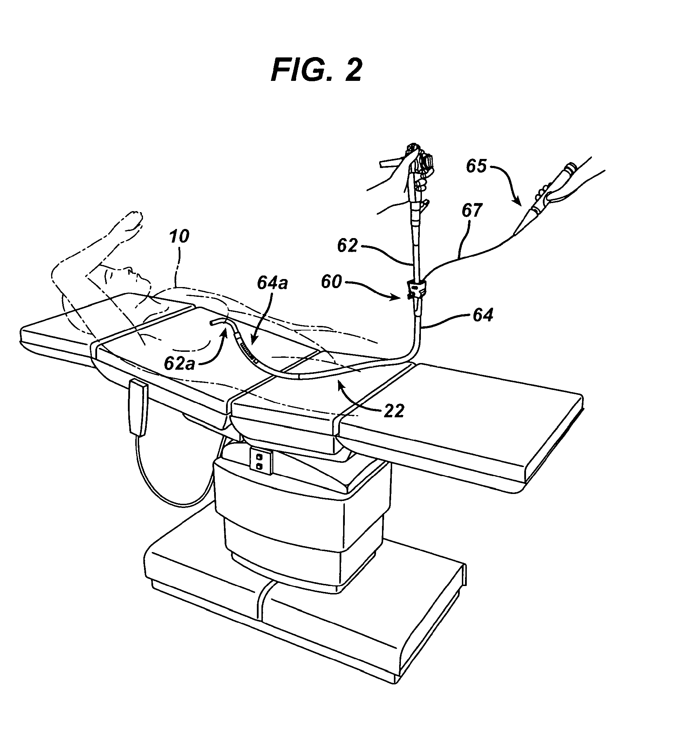

As shown in FIG. 2, the surgeon can also form a vaginal opening or access hole 22 in a vaginal wall of the patient 10 to create an opening between the vagina and the patient's abdominal cavity to gain access to the abdominal cavity. A scoping device having a viewing element located thereon, e.g., an endoscope 62, can be advanced into the vagina before formation of the vaginal access hole 22, and/or it can be advanced through the vaginal access hole 22 after formation to provide visualization inside the patient's body during the surgical procedure. The vaginal access hole 22 can be formed before or after the abdominal access hole 12 of FIG. 1, but in an exemplary embodiment, the vaginal access hole 22 is formed after the abdominal access hole 12 to allow prior insufflation of the patient's abdominal cavity through the abdominal access hole 12. Before forming the vaginal access hole 22, as will be appreciated by a person skilled in the art, the surgeon can dilate and secure the patient's vaginal opening using a surgical instrument, e.g., a weighted speculum, and/or one or more sutures. The vaginal access hole 22 can have any shape and size, but the vaginal access hole 22 preferably has a diameter of about 18 mm and is configured to allow passage of a surgical instrument, e.g., a trocar, a scoping device, a surgical stapler, a clip applier, etc., having a diameter in a range of about 5 mm to 18 mm.

As will be appreciated by a person skilled in the art, access holes through the abdominal and/or vaginal walls can be formed in any way. In an exemplary embodiment, the surgeon can insert a trocar through the vaginal wall to form the vaginal access hole 22 to create an opening between the vagina and the patient's abdominal cavity. Non-limiting embodiments of a trocar can be found in U.S. Patent Publication No. 2007/0260273 filed on May 8, 2006 and entitled "Endoscopic Translumenal Surgical Systems," which is hereby incorporated by reference in its entirety. An exemplary embodiment of a trocar can include a trocar housing configured to allow a surgical device to pass therethrough, and a trocar sleeve or overtube mated to or extending from the trocar housing. The trocar can also include an obturator configured to pass through the trocar housing and the trocar sleeve. The obturator can have an inner lumen formed therethrough for receiving a scoping device and/or other surgical device therein, and a distal end configured penetrate through tissue. The trocar sleeve can be slidably disposed over the obturator and can function as a placeholder after the trocar is inserted through tissue and the obturator is removed, as discussed further below.

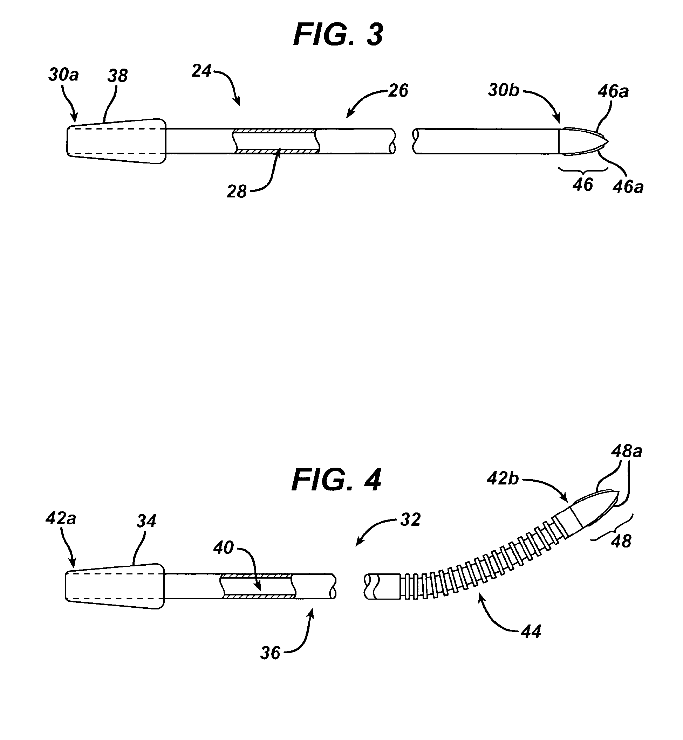

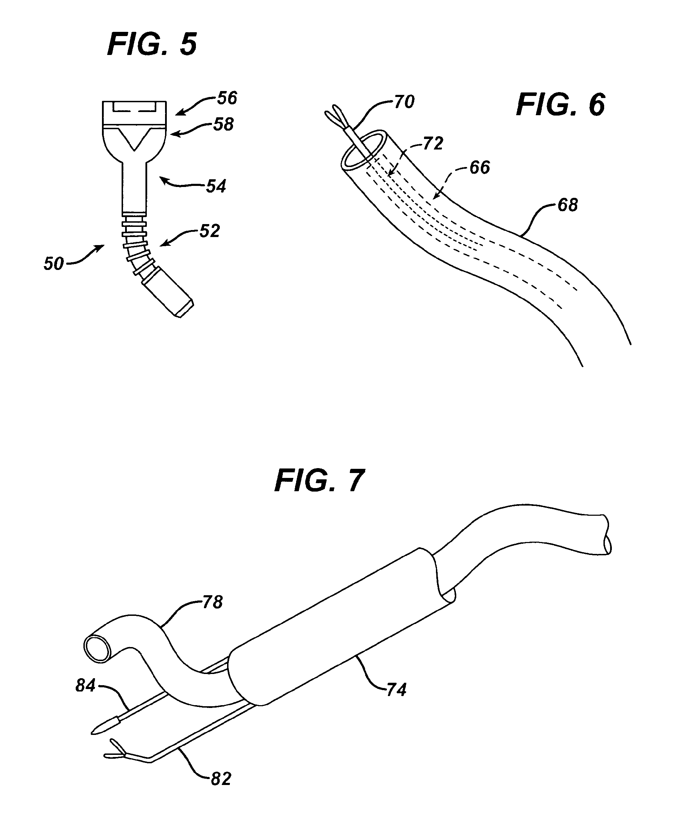

FIGS. 3 and 4 illustrate non-limiting embodiments of obturators. FIG. 3 illustrates a rigid obturator 24 having a proximal handle 38 with an elongated rigid shaft 26 extending therefrom. The rigid shaft 26 has an inner lumen 28 extending between proximal and distal ends 30a, 30b thereof that is configured to receive a surgical device therein. FIG. 4 illustrates a flexible obturator 32 having a proximal handle 34 with an elongated flexible shaft 36 extending therefrom. The flexible shaft 36 has an inner lumen 40 extending between proximal and distal ends 42a, 42b thereof that is configured to receive a surgical device therein. The flexible obturator 32 includes a distal flexible portion 44 configured to cooperate with a flexible portion of an articulating cannulated device through which the flexible obturator 32 can be passed. The articulating cannulated device can include any surgical device having any configuration. FIG. 5 illustrates a non-limiting embodiment of an articulating trocar sleeve 50 that includes a flexible, distal joint 52 on an elongate shaft extending from a rigid proximal housing 54 having a lip seal 56 and a duckbill seal 58 for forming a seal around a surgical device passed therethrough and forming a seal in the inner lumen 28, respectively. The flexible joint 52 can be passively actuated, e.g., moveable when abutted by one or more adjacent structures, and/or actively actuated, e.g., through manipulation of a mechanical and/or manual actuation mechanism. Whether passively or actively actuated, the flexible joint 52 can allow for better positioning of a device inserted through the trocar sleeve 50. The obturator's flexible portion 44 can also be passively and/or manually actuated, though in an exemplary embodiment the flexible portion 44 is passive to allow the obturator 32 to, e.g., navigate through an articulating trocar. The trocar's flexible joint 52 and the obturator's flexible portion 44 can each be formed in any way, same or different from one another, as will be appreciated by a person skilled in the art. For non-limiting example, the trocar's flexible joint 52 and the obturator's flexible portion 44 can be made from a flexible material, can include one or more features formed therein to facilitate flexibility, e.g., a plurality of cut-outs, slots, etc., and/or can be formed from a plurality of linkages that are movably coupled to one another. The articulating trocar sleeve 50 can have any size, but in an exemplary embodiment, the articulating trocar sleeve can be configured to be disposed in a percutaneous opening, e.g., having a diameter not greater than about 5 mm.

Referring again to FIGS. 3 and 4, distal tips 46, 48 of the obturators 24, 32, respectively, can be tapered to facilitate penetration of the tips 46, 48 through tissue. As illustrated, the tips 46, 48 can include end caps with tissue separators 46a, 48a formed on opposed sides thereof and extending between proximal and distal ends of the tips 46, 48. The separators 46a, 48a can protrude outward from an outer surface of their respective tips 46, 48 and can penetrate tissue. The separators 46a, 48a can also be configured to couple to an energy source to facilitate cutting or separating of tissue. For example, a cautery wire can be coupled to a separator, and it can extend through a scoping device attached to the end cap to allow a proximal end of the wire to connect to an energy source. In another embodiment, the separators 46a, 48a can be in the form of paddles that do not cut tissue but rather merely extend outward from an outer surface of their respective tips 46, 48. The paddles can have a generally planer, elongate configuration, and in use they can be configured to penetrate tissue with or without the tissue having a previously formed cut or slit therein, e.g., formed with a cutting instrument. For example, the paddles can be rotated to spread open an elongate cut made through tissue. The separators 46a, 48a can also be used to spread apart tissue and/or to facilitate enlargement of a puncture hole formed through tissue. A person skilled in the art will appreciate that the separators 46a, 48a can be formed integrally with their respective tips 46, 48, such that the tip and separators are formed as a single piece of material, or they can be separate from and mated to their respective tips 46, 48. The tips 46, 48 can taper distally to facilitate insertion and penetration through tissue. The tips 46, 48 can optionally include an opening at their distal-most ends, which can allow a cutting instrument, e.g., a needle knife, a scalpel, a hook knife, etc., to be advanced therethrough to cut tissue and/or a surgical device such as a scoping device to be advanced therethrough.

The distal tips 46, 48 of the obturators 24, 32, respectively, can be made from an optically clear material to allow a scoping device having a viewing element located thereon to see through the distal tip when the viewing element is appropriately positioned near the distal tip. An optically clear distal end is preferably closed, e.g., not in communication with the inner lumens 28, 40, to prevent fluid and/or other debris from contacting the scoping device disposed therein. In other embodiments, the distal tips 46, 48, can be made from an opaque or otherwise non-optically clear material. If the obturator's distal end is non-optically clear, the distal end preferably includes an opening to allow a surgical device such as a scoping device to see and/or be advanced therethrough and/or an endoscopic accessory, e.g., a needle knife, a scalpel, etc., to be advanced therethrough to cut tissue. The distal tips 46, 48 can be integrally formed with the shafts 26, 36, respectively, or the distal tips 46, 48 can be configured as end caps attached to the shafts 26, 36.

As mentioned above, the trocar used to form the vaginal access hole 22 can include a sleeve. While the sleeve can have virtually any configuration, it preferably includes a hollow, elongate flexible shaft that is configured to be slidably disposed over an obturator. The size of the flexible shaft of the trocar sleeve can vary, but it preferably has a length that is slightly less then a length of the shaft of the obturator such that the distal tip of the obturator can extend distally beyond a distal end of the elongate shaft. The shaft's diameter can also vary, but as indicated above, the diameter should be sufficient to allow the elongate shaft of the trocar sleeve to receive the elongate shaft of the obturator therein. The elongate shaft of the trocar sleeve can be rigid or flexible. In one embodiment, the trocar sleeve is a flexible sleeve having a coiled wire wrapped there around or embedded therein to prevent kinking, and having a slipping interior lining to facilitate smooth passage of an obturator therethrough. The elongate shaft of the trocar sleeve can also include regions that vary in flexibility.

The trocar sleeve can also include other features to facilitate use of the trocar sleeve with an obturator. For example, the distal end of the trocar sleeve can have an outer diameter that tapers distally to form a substantially smooth continuous transition from the trocar sleeve to the distal tip of the obturator. The sleeve's distal end can be angled, or it can have various other configurations. In other exemplary embodiments, the sleeve's distal end can be optically clear to facilitate viewing therethrough. The trocar sleeve can also including a housing formed on or coupled to a proximal end of the sleeve. The housing can be configured to removably mate to a handle or a housing of an obturator. The sleeve's housing can also include an inner lumen formed therethrough and coaxial with a lumen in the sleeve's shaft to allow the elongate shaft of the obturator to be inserted through the sleeve's housing and into the sleeve's elongate shaft. One or more seals can be disposed within the lumen in the sleeve's housing to seal the sleeve's shaft and/or to engage an outer surface of the shaft of the obturator to seal the obturator's shaft with respect to the trocar sleeve housing. Any seal can be used, as will be appreciated by a person skilled in the art, such as duck bill or double duck bill valves, iris seals, zero-closure valves, gaskets, etc. A person skilled in the art will also appreciate that the sleeve's housing can include various other features known in the art and that the housing can have virtually any shape and size. Alternatively, the trocar sleeve does not need to include any housing and can merely be in the form of an elongate shaft which can optionally include a locking mechanism, such as a luer lock, for mating to and forming a seal about an obturator disposed therein.

The sleeve can optionally include one or more flexible joints at fixed locations along the sleeve's longitudinal length. The flexible joint(s) can be located anywhere along the sleeve's longitudinal length, but at least one flexible joint is preferably located in a distal portion of the sleeve so the sleeve can articulate near a location where a surgical instrument extends out of the sleeve into a patient's body. The sleeve can actively and/or passively articulate in the flexible joint(s) to help maneuver the sleeve around potential obstructions, e.g., tissue, other surgical instruments, etc., in the body and to help direct surgical devices advanced through the sleeve to a more desirable surgical location in the body. The flexible joint(s) can be similar to the trocar flexible joint 52 and the obturator flexible portion 44 described above and can be formed in any way, as will be appreciated by a person skilled in the art. A person skilled in the art will appreciate that flexible joints can also pivot or more about a single point, rather than flexing along a length of the shaft.

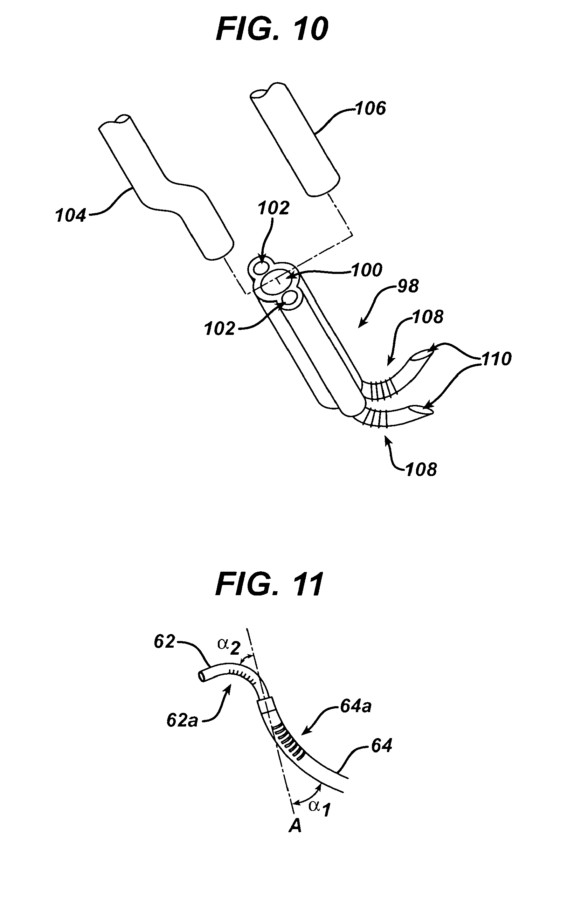

In an exemplary embodiment, the sleeve can be a standalone sleeve. A standalone sleeve can be used as a standalone device inserted through an access hole, or it can be placed alongside or through a trocar shaft. A standalone sleeve can allow a plurality of tools to be inserted therethrough. A standalone sleeve can optionally include one or more flexible joints along its longitudinal length as discussed above. FIG. 6 illustrates one embodiment of a flexible standalone sleeve, FIGS. 7-10 illustrate exemplary embodiments of rigid standalone sleeves, and FIG. 11 illustrates a standalone sleeve that can be rigid or flexible.

As shown in FIG. 6, a flexible standalone sleeve 66 can include an elongate flexible shaft configured to be disposed through an access hole in a body. One or more flexible surgical instruments, e.g., a grasper 70 as shown, can be passed through an inner pathway 72 of the flexible standalone sleeve 66. A person skilled in the art will appreciate that the term "grasper" as used herein is intended to encompass any surgical instrument that is configured to grab and/or attach to tissue and thereby manipulate the tissue, e.g., forceps, retractors, movable jaws, magnets, adhesives, stay sutures, etc.

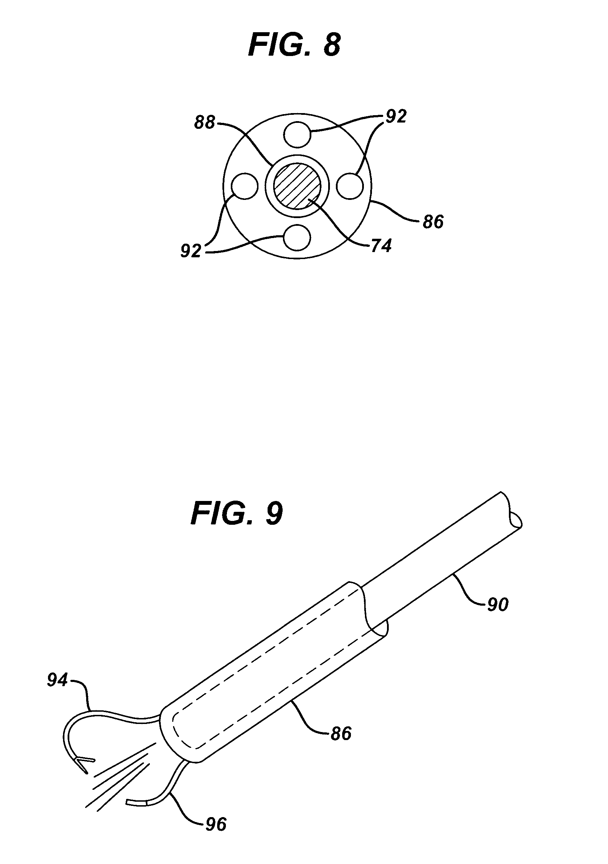

A rigid sleeve can provide rigidity to one or more flexible surgical devices advanced through the rigid sleeve while allowing the flexible surgical device(s) to flex outside the rigid sleeve. FIGS. 7 and 8 show one embodiment of a rigid sleeve 74 having an inner pathway 88 extending therethrough through which a surgical instrument, e.g., a flexible scoping device 90, can be passed. The rigid sleeve 70 can include supplemental instrument passages 92 extending therethrough. Four supplemental passages 92 are illustrated, but the rigid sleeve 74 can include one or more supplemental passages. The supplemental passages 92 can have any configuration in the rigid sleeve 74, e.g., equidistantly and circumferentially located around the flexible inner pathway 76 as shown. A rigid or flexible surgical instrument, e.g., a rigid pair of movable jaws 82, a rigid tissue retractor 84, etc., can be passed through any one of the supplemental passages 92, which can be flexible or rigid in any combination. The supplemental passages 92 can thus allow multiple surgical instruments to be selectively advanced into a body through a single port or access hole through which the rigid sleeve 74 is disposed. The scoping device 78 can work in cooperation with device(s) advanced through any one or more of the supplemental passages 92 to provide visualization of the device(s), where the flexibility of the scoping device 78 can improve the angle of visualization of the device(s) and allow the scoping device 78 to flex away from the operative site to help reduce interference of the scoping device 78 with operation of the device(s) in the body.

FIG. 9 illustrates an alternate embodiment of a rigid sleeve 86 that includes an inner pathway, similar to the inner pathway 88 described above, through which a surgical instrument, e.g., a rigid scoping device 90, can be passed. The alternate rigid sleeve 86 can include one or more supplemental instrument passages extending therethrough, similar to the supplemental passages 92 described above. The rigid sleeve's inner pathway can provide a constant, continuous orientation of the scoping device 90 relative to flexible or rigid surgical instruments, e.g., a flexible pair of movable jaws 94, a flexible tissue retractor 96, etc., advanced through the passage(s), while flexible instrument(s) advanced through the supplemental passages can be maneuvered to an increased oblique angle with respect to the scoping device 90.

FIG. 10 shows another embodiment of a standalone sleeve 98, including a central inner pathway 100 extending therethrough and a pair of side inner pathways 102 also extending therethrough and located on opposed sides of the central pathway 100. The standalone sleeve 98 is shown as rigid, but it can be rigid or flexible. Generally, the central pathway 100 can be configured to receive a flexible scoping device 104 or a rigid scoping device 106 disposed therein, while the side pathways 102 can each be configured to receive at least one surgical instrument disposed therein. Two side pathways 102 are illustrated, but the standalone sleeve 98 can include any number of side pathways. One or more of the side pathways 102 can include a flexible or articulating arm 108 extending from a distal end thereof and having an inner pathway 110 in communication with its respective side pathway 102. The articulating arm(s) 108 can be configured as passively and/or actively articulated flexible joints, similar to those discussed above, to allow surgical devices disposed therein to bend for more advantageous orientation in a body.

Referring again to FIG. 2, a trocar used to form the vaginal access hole 22 can include a trocar sleeve or overtube 64 having a trocar housing 60 at its proximal end through which one or more surgical devices can be inserted. An obturator, e.g., the rigid obturator 24 of FIG. 3 or the flexible obturator 32 of FIG. 4, can be advanced through the sleeve 64 to help form the vaginal access hole 22. The sleeve 64 can optionally be guided through the body using a steering mechanism on the sleeve 64, on the endoscope 62 disposed in the sleeve 64, or using other techniques known in the art. Following formation of the vaginal access hole 22, the obturator can be removed from the sleeve 64. The sleeve 64 can remain in the patient 10 and function as a placeholder for the vaginal access hole 22, as the sleeve 64 can extend through the vaginal access hole 22 and into the abdominal cavity. The endoscope 62 can be removed from the obturator (or, for the embodiment of an obturator in the form of a scoping device having an end cap, the end cap can be removed from the scoping device). The endoscope 62 can be reinserted through the trocar sleeve 64, as shown in FIG. 2. The trocar sleeve 64 can remain in place or it can be removed leaving the endoscope 62 in place through the vaginal access hole 22.

As shown, the sleeve 64 is preferably left in the patient 10 at least because the illustrated sleeve 64 includes a steerable distal end having at least one distal flexible joint 64a that can help orient the endoscope 62 (and/or any other device advanced through the sleeve 64) as discussed above. The sleeve's flexible joint 64a can be actuated using an actuation device 65 connected to the flexible joint 64a via an actuation cable 67, although a person skilled in the art will appreciate that the flexible joint 64a can be actuated in this or any other way. The flexible joint 64a can be configured to bend in a single direction when actuated using the actuation cable 67, and the single direction can be selectively chosen, e.g., left, right, up, down, etc., via the actuation device 65. If the sleeve 64 includes a plurality of flexible joints, each of the flexible joints can be configured to be independently actuated in any direction same or different from any of the other flexible joints of the sleeve 64. The actuation device 65 can be configured to control the amount of movement in a chosen direction. Optionally, the endoscope 62 can, additionally or alternatively to the sleeve's flexible joint 64a, have at least one of its own distal, flexible joints 62a. The endoscope's flexible joint 62a can be actuated in any way, as can any additional flexible joints of the endoscope 62. Thus, as shown in FIG. 11, the sleeve's flexible joint 64a can bend the sleeve 64, and also the endoscope 62 disposed therein, at a first location along a longitudinal axis A of the endoscope 62 in a first direction at a first angle α₁ relative to the endoscope's longitudinal axis A, while the endoscope's flexible joint 62a can bend the endoscope 62 at a second location along the endoscope's longitudinal axis A in a second direction at an a second angle α₂ relative to the endoscope's longitudinal axis A. The first and second angles α₁, α₂ can be chosen to position the distal end of the endoscope 62 in any desired way, e.g., in an "S" shape as illustrated. Being configured to have an "S" shape, the endoscope 62 can be vaginally introduced into the patient 10 yet provide a view of the patient's stomach in a direction toward a fundus of the stomach similar to a view that could be provided by a scoping device inserted into the abdominal cavity through an incision in the abdomen 14.

In an alternate embodiment, a scoping device can have two or more flexible joints each at different locations along its longitudinal axis to allow the scoping device, with or without use of a sleeve, to bend in at least two directions relative to the scoping device's longitudinal axis. A non-limiting example of a multibending scoping device is the R-Scope XGIF-2TQ260ZMY available from Olympus Corp. of Tokyo, Japan.

Optionally, the endoscope 62 advanced into the patient 10 can be mounted to a support external to the patient 10 to allow hands-free use. The endoscope 62 can be mounted at any time, and its mounting can be re-adjusted and/or released at any time, but in an exemplary embodiment, the endoscope 62 is mounted following arrangement of the endoscope's viewing element at a desired location in the patient 10. By mounting the endoscope 62, the surgeon does not need to continuously hold the endoscope 62 in place during the surgical procedure, thereby freeing the surgeon to attend to other surgical matters, and/or reducing the required number of operating room personnel. Stably mounting a scoping device with a viewing element located thereon can also help stabilize images acquired through the viewing element. A person skilled in the art will appreciate that a support can be used to mount the endoscope 62 and/or any other surgical instrument used during the gastrectomy that does not require constant hands-on manipulation. Multiple supports can be used in a single surgical procedure.

The support can have a load limit considering forces such as a weight of the mounted surgical instrument and forces from hand-placing the mounted surgical instrument. The support's load limit can be predetermined, e.g., as limited by a gooseneck and/or other component of the support, or it can be adjustable, e.g., using a central tension cable having movable concave and/or convex ends, a plate with gear faces, different Vlier pins, etc.

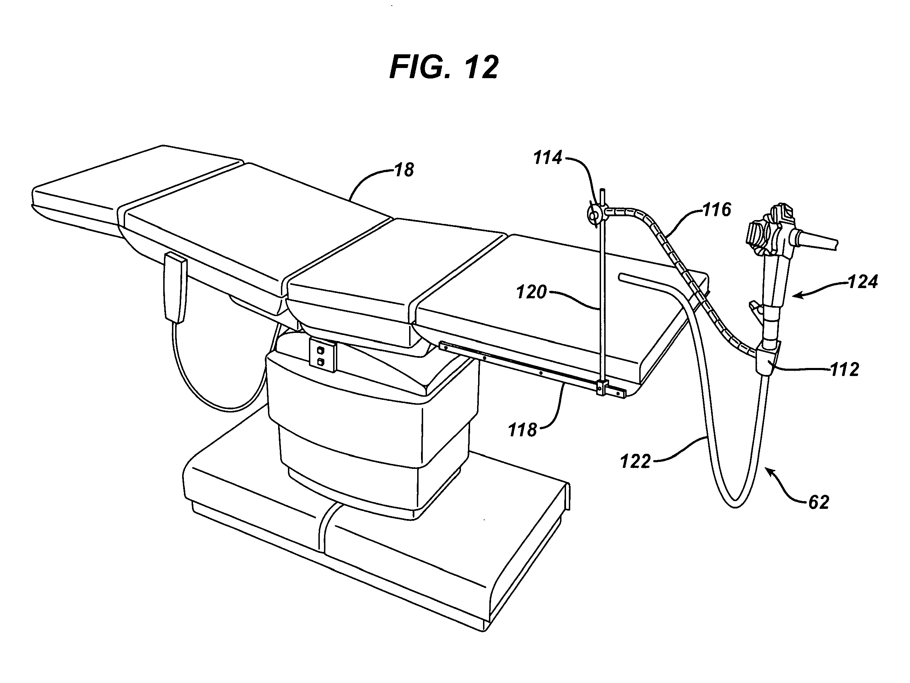

The support can have a variety of configurations, and FIG. 12 shows one embodiment of a support in use to mount the endoscope 62 to the examination table 18. The illustrated support includes a scope holder 112, an adapter 1 14, and a flexible arm 116 coupled at its respective terminal ends to the scope holder 112 and the adapter 114. The adapter 114 can mate, as shown, to a table mount coupled to the examination table 18 and including a table rail 118 and a bracket 120 coupled at its respective terminal ends to the table rail 118 and the adapter 114. In an alternate embodiment, in addition to or instead of the examination table 18, the support can mount to another stable structure near the patient 10, e.g., a wall, the ceiling, an independent structure standing on the floor similar to an IV pole or a microphone stand, an overhead fixture, etc.

The scope holder 112 is generally configured to engage the device being mounted. As shown, the scope holder 112 includes a block having a bore extending therethrough which can receive and hold the endoscope 62. The bore's diameter can be large enough to allow passage of a shaft 122 of the endoscope 62, inserted distal end first into the bore, and can be small enough to prevent passage of a proximal handle 124 of the endoscope 62 to thereby hold the endoscope 62. Different scope holders having different size bores can be selectively attached to the flexible arm 116 to allow surgical instruments of different sizes to be mounted using the support. Alternatively, the diameter of the scope holder's bore can be adjustable.

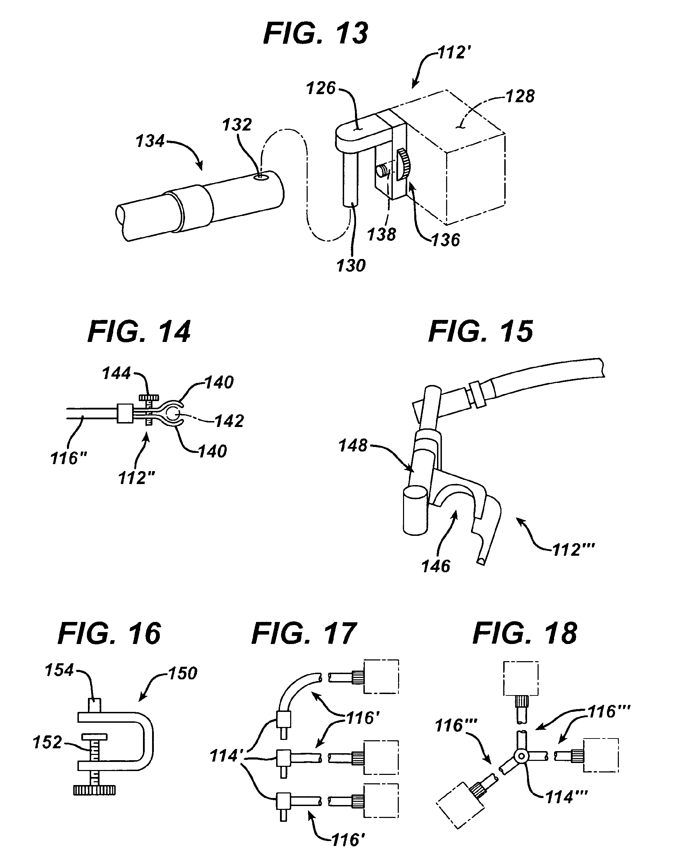

FIG. 13 shows an alternate embodiment of a device holder 112' that includes a holder adapter 126 configured to mount to a holder block 128 having a bore therethrough for receiving a surgical device. The holder adapter 126 and the block 128 can be attached together in any way, e.g., using one or more screws. The holder adapter 126 can have a peg 130 extending therefrom that is configured to engage a bore 132 formed in an adjustment bar 134. The holder adapter 126 can rotate around a longitudinal axis of the peg 130 and can be fixed in a desired position by turning a thumbwheel 136 coupled to the holder adapter 126, which can advance a post 138 coupled to the thumbwheel 138 to contact the adjustment bar 134 and fix the holder adapter 126 in place with respect to the adjustment bar 134. The adjustment bar 134 can optionally have a cannulated interior into which one or more surgical device cords can be fed to help keep the cords from interfering with the surgical procedure.

FIG. 14 shows another alternate embodiment of a device holder 112". The scope holder 112" includes a pair of opposed clamping arms 140 coupled to a flexible arm 116". A surgical device 142 can be positioned between device-contacting surfaces of the clamping arms 140, and the clamping arms 140 can be tightened together to secure the device 142 therebetween by, e.g., turning a tightening screw 144. The clamping arms 140 can each have a convex, device-contacting surface to help grip a cylindrical device, e.g., a shaft of a scoping device.

FIG. 15 shows yet another alternate embodiment of a device holder 112"'. The device holder 112"' includes a first semi-cylindrical recess 146 for receiving a surgical device to be mounted, e.g., a cylindrical device shaft, and a second semi-cylindrical recess 148 for receiving a handle, a cord, and/or other proximal extension from the mounted device. The second recess 148 can be configured to act as a stop mechanism or rest which can hold the device in place, while the first recess 146 can cooperate with the second recess 148 to help prevent the mounted device from pivoting in the device holder 112"'. The first and second recesses 146, 148 are each shown as having a semi-cylindrical shape, but as will be appreciated by a person skilled in the art, the recesses 146, 148 can have any shape, same or different from one another, to accommodate any device.

Referring again to FIG. 12, the flexible arm 116 is generally configured to be movable, as will be appreciated by a person skilled in the art, to allow the mounted device's position to be adjusted relative to the examination table 18. The flexible arm 116 can have a fixed or adjustable longitudinal length. The flexible arm's longitudinal length can be made adjustable in any way appreciated by a person skilled in the art, e.g., having one or more flexible joints, being accordion-expandable, etc.

The adapter 114 is generally configured to secure the flexible arm 116 to the bracket 120 and hence to the table 18. The adapter 114 can be movable, e.g., axially movable along the bracket 120 and/or rotatably movable around a longitudinal axis of the bracket 120, to increase possible mounted positions of the endoscope 62.

The table mount including the bracket 120 and the table rail 118 are each generally configured as rigid elongate bars that can provide secure attachment of the flexible arm 116 to the table 18. The bracket 120 and the table rail 118 can have fixed lengths, or at least one of them can be configured to be expandable in length. FIG. 16 illustrates an alternate embodiment of a table mount that can directly couple to a flexible arm. A c-clamp 150 can mount to the examination table 18 (or any other stable structure) by positioning the table 18 within the "c" and tightening the "c" using, e.g., a hand-turned screw clamp 152. A flexible arm can couple to the c-clamp 150 at an arm connector 154 of the c-clamp 150, e.g., with a disengagable metallic ball coupling, thereby securely mounting the flexible arm and any surgical device(s) coupled to it.

In some embodiments, a single support can be configured to mount multiple surgical instruments, such as by using an adapter configured to secure multiple flexible arms coupled to multiple adapters to mount multiple devices. FIG. 17 shows one embodiment of a multi-arm adapter that includes at least two stackable adapters 114' configured to stack on top of one another such that the stackable adapters 114' are axially aligned. The stackable adapters 114' can rotate around a longitudinal axis of the adapters 114' so a device can be held in each one of the adapters' respective holder blocks and be movably adjusted using their respective flexible arms 116'. FIG. 18 shows another embodiment of a multi-arm adapter 114" that includes at least two radial flexible arms 116"' extending tangentially therefrom. The radial arms 116" can be movable, e.g., rotated around a longitudinal axis of the adapter 114", to position the arms 116"' as desired.

During the surgical procedure, the patient's stomach can be difficult to adequately access. The patient's liver can be retracted during the gastrectomy to help the surgeon gain better access to the stomach. Although the liver can be retracted at any time during the surgical procedure, in an exemplary embodiment the liver is retracted after insertion into the patient 10 of a scoping device having a viewing element located thereon, e.g., the endoscope 62 through the vaginal access hole 22, to provide visualization of the abdominal cavity before and during retraction of the liver. Although visualization before, during, and/or subsequent to liver retraction can be provided using a scoping device that is introduced into the abdominal cavity through an opening in the abdominal wall 14, providing visualization with a vaginally introduced scoping device can allow for increased abdominal work space and/or reduce the "chopstick" effect of abdominally introduced instruments. The liver can be retracted in any way appreciated by a person skilled in the art, but the liver is preferably retracted using at least one device inserted into the abdominal cavity of the patient 10 through, e.g., the previously-formed abdominal access hole 12, through another abdominal opening, through an access hole in a wall of a digestive tract of the patient, etc. Also as will be appreciated by a person skilled in the art, a draining device, e.g., a penrose drain, a Jackson-Pratt drain, etc., can be disposed in the patient's abdominal cavity to help hold the liver and/or drain excess fluid that can accumulate in the abdominal cavity during the surgical procedure, particularly following liver retraction.

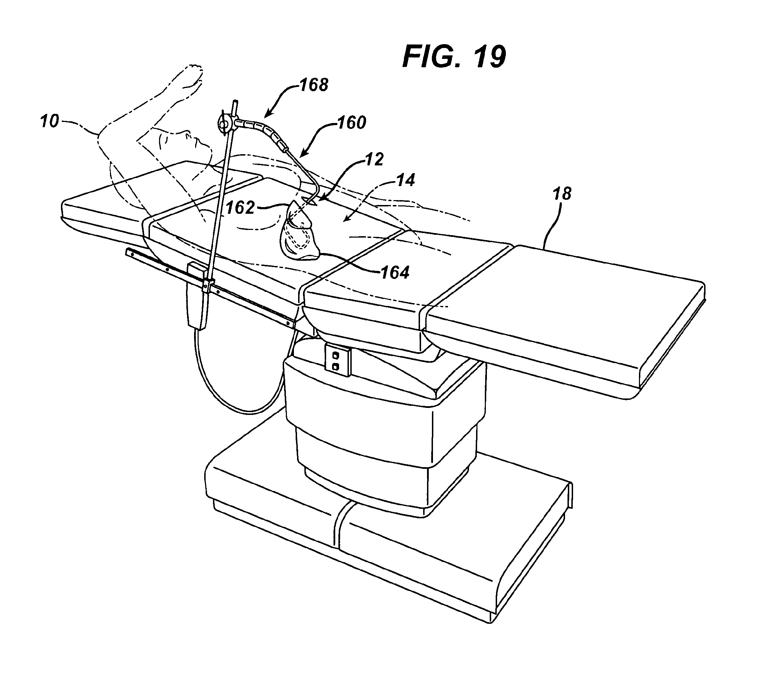

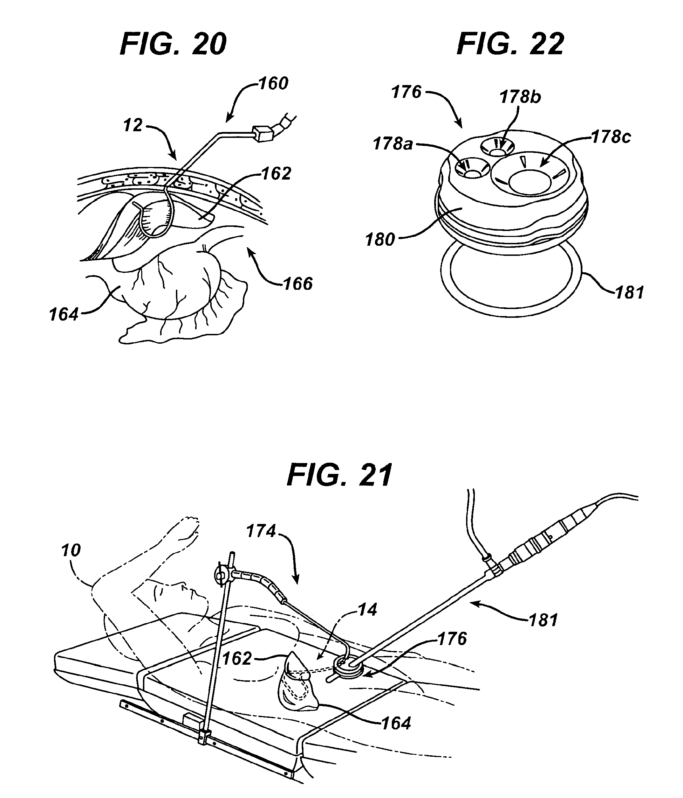

A retractor device, such as a Nathanson liver retractor, can be used to retract the patient's liver. FIGS. 19 and 20 illustrate one embodiment of a liver retraction procedure using a Nathanson liver retractor 160 to retract a liver 162 of the patient 10 away from a stomach 164 of the patient 10. As will be appreciated by a person skilled in the art, the surgeon can use the Nathanson liver retractor 160 to "hook" the liver 162 as shown in FIG. 19 and hold the liver 162 away from the stomach 164 in a desired retracted position as shown in FIG. 20. A support 168 similar to the support of FIG. 12 can be used to mount the Nathanson liver retractor 160 to the examination table 18, although any other support can be used if a support is used at all for a liver retractor. The Nathanson liver retractor 160 can be directly inserted through the abdominal access hole 12 as illustrated, or the Nathanson liver retractor 160 can be advanced through a cannulated device providing access into the patient's abdominal cavity 166, e.g., through the trocar 16, through a multiple port access device, through a sleeve, etc.

FIG. 21 illustrates an alternate embodiment of a liver retraction procedure using a Nathanson liver retractor 174 and a multiple port access device 176 positioned in the patient's abdominal wall 14 at an umbilicus of the patient 10. The multiple port access device 176 can be positioned in the abdominal access hole 12 or in another abdominal access hole, which in an exemplary embodiment includes a percutaneous opening. Various non-limiting embodiments of a multiple port access device can be found in U.S. Patent Publication No. 2006/0247673 filed April 5, 2006 and entitled "Multi-port Laparoscopic Access Device," U.S. Application No. [ ] entitled "Surgical Access Device" [Atty. Docket No. 100873-310 (END6485USNP)] and filed on even date herewith, U.S. Application No. [ ] entitled "Surgical Access Device with Protective Element" [Atty. Docket No. 100873-31 (END6485USNP1)] and filed on even date herewith, U.S. Application No. [ ] entitled "Multiple Port Surgical Access Device" [Atty. Docket No. 100873-312 (END6485USNP2)] and filed on even date herewith, and U.S. Application No. [ ] entitled "Variable Surgical Access Device" [Atty. Docket No. 100873-313 (END6485USNP3)] and filed on even date herewith, which are hereby incorporated by reference in their entireties. The multiple port access device 176 can have any size, shape, and configuration, but in an exemplary embodiment shown in FIG. 22, the multiple port access device 176 has a housing 180 with three ports 178a, 178b, 178c extending therethrough, although the multiple port access device 176 can have any number of ports. In addition, the ports 178a, 178b, 178c can have the same size or varying sizes configured to provide for the insertion of differently sized surgical instruments therethrough. In an exemplary embodiment, the ports 178a, 178b, 178c can each be sized to provide for instruments less than or equal to about 5 mm in diameter by having diameters of about 5 mm, preferably with at least one of the ports, e.g., the smaller ports 178a, 178b as illustrated, having a diameter of about 3 mm. The ports 178a, 178b, 178c can each include one or more sealing ports configured to provide a seal to prevent the escape of insufflation gas and/or to form a seal around an instrument inserted therethrough. The housing 180 can also include a flexible retractor 181 extending distally therefrom and configured to be positioned within an opening, e.g., an otomy, in tissue to form a pathway through the tissue for instruments inserted through the ports 178a, 178b, 178c. The multiple port access device 176 can also be used to form a vaginal access hole for introducing multiple devices into the abdominal cavity through the vagina.

Referring again to FIG. 21, the patient's liver 162 can be retracted using the Nathanson liver retractor 174 similar to the liver retraction procedure of FIGS. 19 and 20 using the Nathanson liver retractor 160. In this embodiment, however, the Nathanson liver retractor 174 can be inserted into the patient 10 through one of the smaller ports 178a, 178b in the multiple port access device 176. The patient's abdominal cavity can be visualized using the endoscope 62 advanced through the vaginal access hole 22 or, as shown, a scoping device having a viewing element located thereon, e.g., a laparoscope 181, can be advanced through one of the multiple port access device's ports 178a, 178b, 178c, e.g., the larger of the ports 178c. With visualization provided by the laparoscope 181, the endoscope 62 need not be advanced through the vaginal access hole 22 (or can be removed from the vaginal access hole 22 if already advanced therethrough). Further, a grasper (not shown) can be advanced through the vaginal access hole 22, e.g., directly, through a working channel of the endoscope 62, through a multiple port access device positioned vaginally, etc., to assist in retracting the liver 162 and/or otherwise assist in the gastrectomy.

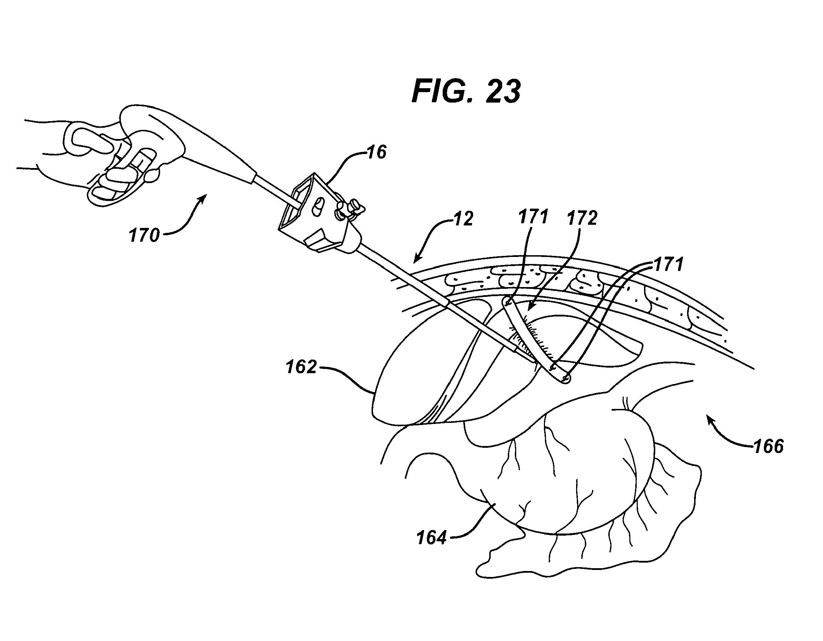

FIG. 23 illustrates an alternate embodiment of a liver retraction procedure that uses a tacker device 170 to help retract the patient's liver 162 to a desired location away from the patient's stomach 164. As will be appreciated by a person skilled in the art, the tacker device 170 can deliver and apply one or more tacks 171 and/or mesh to the abdominal cavity 166 to lift and support the liver 162. FIG. 23 shows tacks 171 applying a penrose drain 172 to help drain fluid away from the surgical site, which in this embodiment can also serve as a retractor device to help hold the liver 162 in its desired retracted position. The tacker device 170 can be inserted through one of the ports 178a, 178b, 178c in the multiple port access device 176 or through another access hole in the abdomen.

In another embodiment, the surgeon can introduce into the patient 10 suture anchors, e.g., t-tags, hooks, etc., having sutures attached thereto. The sutures can be attached to the liver 162, tensioned to desirably position the liver 162, and extracorporeally tied or otherwise secured to maintain the liver 162 in a desired position. In still another embodiment, the liver 162 can be retracted using magnets. The surgeon can affix one or more internal magnets to the liver 162 and one or more external magnets on an outside surface of the patient's abdomen wall 14. The external magnets can attract the internal magnets, thereby moving the liver 162 toward an inner surface of the abdominal wall 14. A liver retracting device can be used alone or in combination with any one or more other liver retracting devices, e.g., magnets in combination with tackers and mesh, a Nathanson liver retractor in combination with suture anchors and sutures, a Nathanson liver retractor in combination with a surgical adhesive, etc.

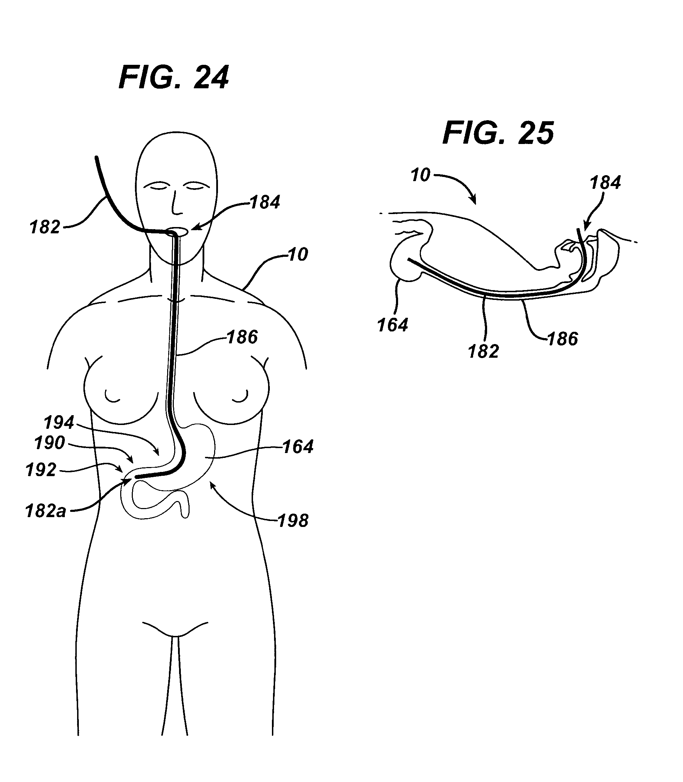

With the patient's stomach 164 accessible and visible to desired degrees, the surgeon can manipulate the stomach 164 to form a gastric tube or stomach sleeve, which can be full or partial, in the stomach 164. As illustrated in FIGS. 24 and 25, the surgeon can introduce a sizing device 182 into the stomach 164 to help size the portion of the stomach 164 that will form the stomach sleeve. The sizing device 182 can be introduced into the stomach 164 in any way, but in this illustrated exemplary embodiment, the sizing device 182 is transorally introduced into the stomach 164, e.g., through a mouth 184 and an esophagus 186 of the patient 10. A person skilled in the art will appreciate that the term "sizer," "sizing device," or "sizing instrument" as used herein is intended to encompass any surgical instrument that is configured to indicate a desired gastric sleeve area, e.g., a bougie, a scoping device, a catheter, etc. The sizer 182 can optionally include a light at its distal end to help the surgeon advance the sizer 182 through the esophagus 186 and desirably position the sizer 182 in the stomach 164. The sizer's size and shape can generally correspond to a size and shape of the stomach sleeve desired to be formed in the patient 10, so the surgeon can choose a sizer having any size, shape, and configuration that generally corresponds to the desired stomach sleeve dimensions. In an exemplary embodiment, the sizer 182 includes a flexible surgical instrument having a substantially cylindrical shape and a substantially constant diameter along the sizer's longitudinal length in the range of about 28 to 42 French (about 9.3 to 14 mm).

The surgeon can movably adjust the sizer 182 in the stomach 164 to place the sizer 182 in a sizing position that generally indicates the size and position of the stomach sleeve following at least partial transection of the stomach 164. The sizing position can be chosen by the surgeon, and in an exemplary embodiment, the sizer 182 in the sizing position extends along a lesser curvature 194 of the stomach 164 and into a pylorus 190 of the stomach 164 so at least a distal-most end 182a of the sizer 182 extends to a pyloric sphincter or valve 192 of the pylorus 190. The sizer 182 can be adjusted in the patient 10 in any way, as will be appreciated by a person skilled in the art. In an exemplary embodiment, sizer adjustment can be performed using a flexible grasper inserted into the stomach 164 through the vaginal access hole 22 for adjusting the sizer 182. The grasper can include an end effector having two opposed, movable jaws configured to grasp and move the sizer 182 once the sizer 182 has been adequately advanced into the patient 10. The grasper can be advanced through a working channel of the endoscope 62 inserted through the vaginal access hole 22. The endoscope 62 can have a light located thereon which can help the surgeon find and grasp the sizer 182 with the grasper and to locate the pyloric valve 192. Alternatively or in addition, a scoping device inserted through the patient's abdominal wall 14 can provide visualization and/or light during this, or any other portion, of the surgical procedure.

The surgeon can measure a distance along a greater curvature 198 of the stomach 164 from the pyloric valve 192 to determine a starting location for transection of the stomach 164. The starting location can be any distance from the pyloric valve 192, but in an exemplary embodiment, the distance is about 6 centimeters. The surgeon can mark the starting location in any way, such as by mentally marking or remembering the starting location or by applying a marker. As will be appreciated by a person skilled in the art, any marker can be used to mark the starting location, e.g., ink applied via a marking device inserted through a vaginal or abdominal access hole, a mark using electrocautery, a mark using a harmonic scalpel, etc. The starting location can be determined before or after introduction of the sizer 182 into the stomach 164 and before or after placement of the sizer 182 in the sizing position.

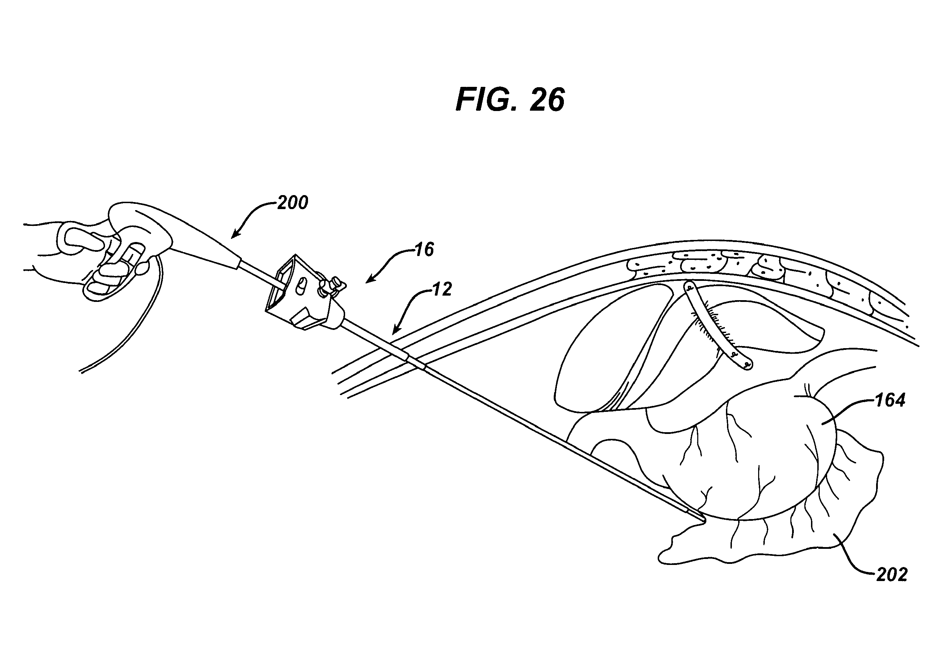

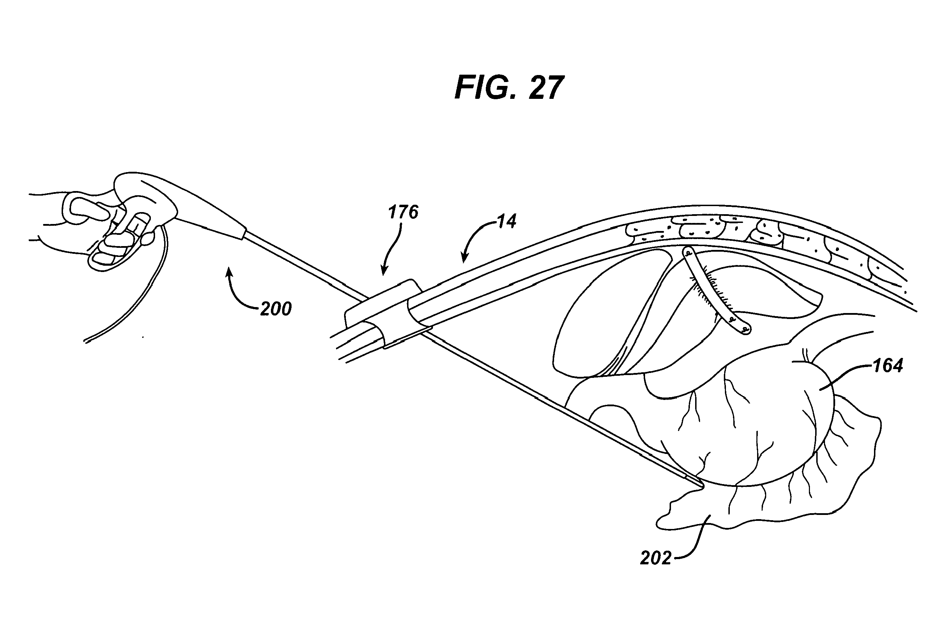

Prior to transecting the stomach 164, the stomach 164 can be separated from tissue attached to the stomach 164, e.g., an omentum, vessels, any adhesions on the stomach 164, etc., to free a fundus of the stomach 164. As will be appreciated by a person skilled in the art, the tissue attached to the stomach 164 can be separated from the stomach 164 using any one or more dissecting devices. A person skilled in the art will also appreciate that the term "dissector," "dissecting device," or "dissecting surgical instrument" as used herein is intended to encompass any surgical instrument that is configured to cut tissue, e.g., a scalpel, a harmonic scalpel, a blunt dissector, a cautery tool configured to cut tissue, scissors, an endoscopic linear cutter, a surgical stapler, etc. Non-limiting embodiments of a dissector having a distal hood can be found in U.S. Patent Application No. [ ] filed on even date herewith and entitled "Methods And Devices For Performing Gastroplasties Using A Multiple Port Access Device," [Atty. Docket No. 100873-319 (END6489USNP)], which is hereby incorporated by reference in its entirety. The desired tissue can be separated from the stomach 164 in any way, but in an exemplary embodiment the surgeon cuts adjacent to the greater curvature of the stomach 164 to free the fundus from the omentum. The dissector can be introduced into the patient 10 through any access hole (natural or surgically created). In one embodiment shown in FIG. 26, a dissector 200 can be inserted through the trocar 16 in the abdominal access hole 12 and can be used to cut an omentum 202 from the stomach 164. As shown in this illustrated embodiment, the dissector 200 has an end effector 200a with a distal end having a pair of movable jaws configured to cut tissue. In an alternate embodiment shown in FIG. 27, the dissector 200 can be inserted through a multiple port access device, e.g., the multiple port access device 176 of FIG. 22, inserted through the patient's abdominal wall 14 and can be used to cut the omentum 202 from the stomach 164.

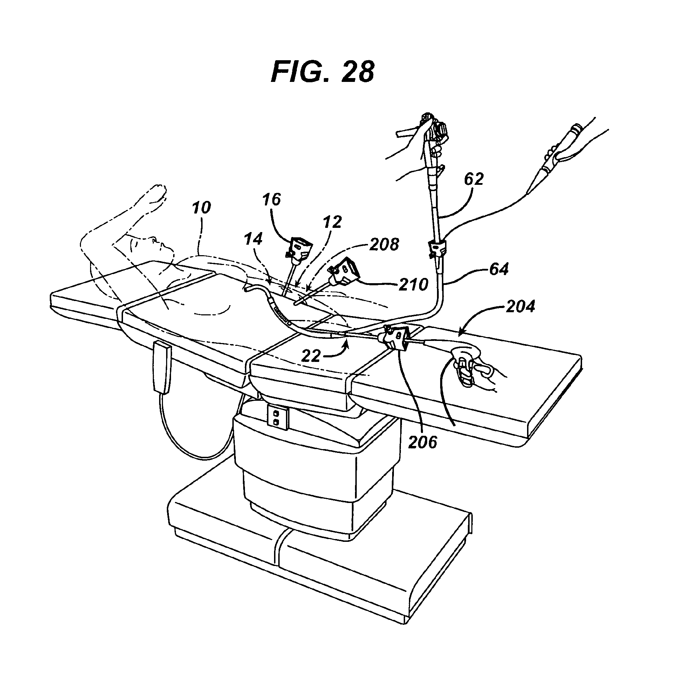

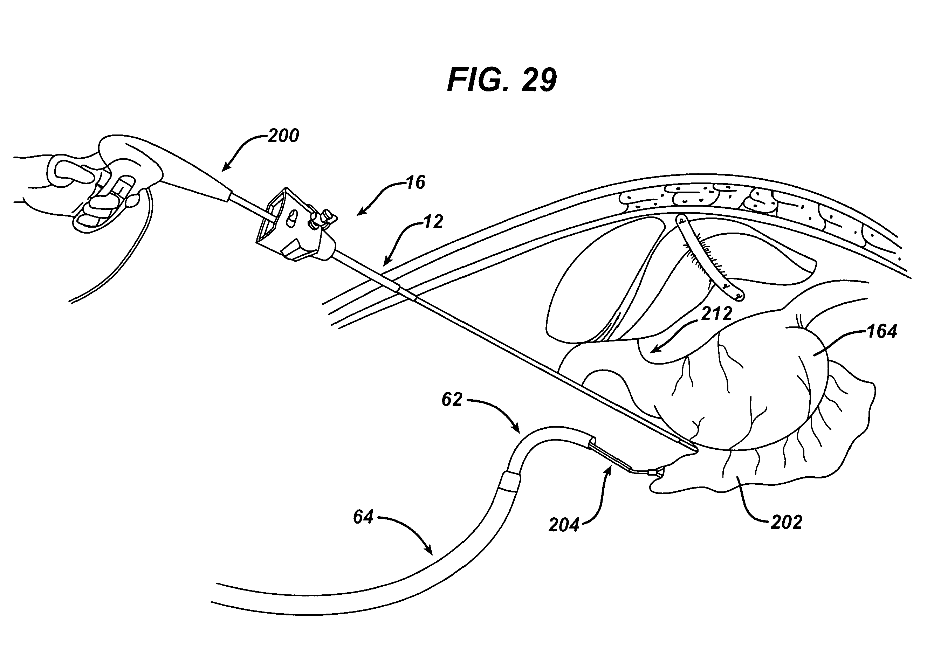

In an exemplary embodiment, the omentum 202 and/or any other desired tissue can be tensioned using a grasper while the dissector 200 dissects the tissue from the stomach 164. The grasper can be introduced into the patient 10 in any way, but as illustrated in an exemplary embodiment shown in FIGS. 28 and 29, the surgeon can advance a grasper, e.g., a grasper 204, through a vaginal trocar 206 inserted through the vaginal access hole 22. Generally, the surgeon can pass tissue from the dissector 200 to the grasper 204, grasp the tissue with the grasper 204, pull the grasper 204 to tension the grasped tissue, and dissect tissue using the dissector 200. The surgeon can repeat this process any number of times to free the stomach fundus. FIG. 28 illustrates a second abdominal access hole 208, e.g., a percutaneous opening, that can be formed using a second trocar 210 similar to that described above regarding the abdominal access hole 12 formed using the trocar 16. The surgeon can insert any one or more desired surgical instruments simultaneously and/or sequentially through the second abdominal access hole 208, with or without the second trocar 210 disposed therein. For non-limiting example only, the surgeon can advance at least one additional grasper through the second abdominal access hole 208 and use the second grasper in cooperation with the grasper 204 inserted through the vaginal trocar 206 to tension the omentum 202. In some embodiments, the surgeon can use only a grasper inserted through the abdominal wall 14, e.g., through the second abdominal access hole 208, and not a vaginally inserted grasper. Alternatively, the surgeon can advance the additional grasper through another access hole, e.g., the vaginal access hole 22 via a working channel of the endoscope 62, through a multiple port access device inserted in an abdominal or vaginal access hole, etc. In some embodiments, one or more graspers for tensioning the dissected tissue can be inserted through the vaginal access hole 22, e.g., through a multiple port access device, and none through the patient's abdomen.

If a scoping device, e.g., the endoscope 62, the laparoscope 20, etc., is inserted in the abdominal cavity, the surgeon can use the scoping device to provide visualization to help position the grasper 204 and/or an additional grasper. The surgeon can position the distal end of the endoscope 62 in any desired way, e.g., in an "S" shape as discussed above and as shown in FIG. 29 to allow visualization of the stomach 164 in a direction toward a fundus 212 of the stomach 164. When the grasper 204 is desirably positioned, e.g., grasping the omentum 202, the scoping device can optionally be removed from the access hole through which is it inserted and the dissector 200 can be inserted therethrough, preferably when another scoping device is present to provide visualization.

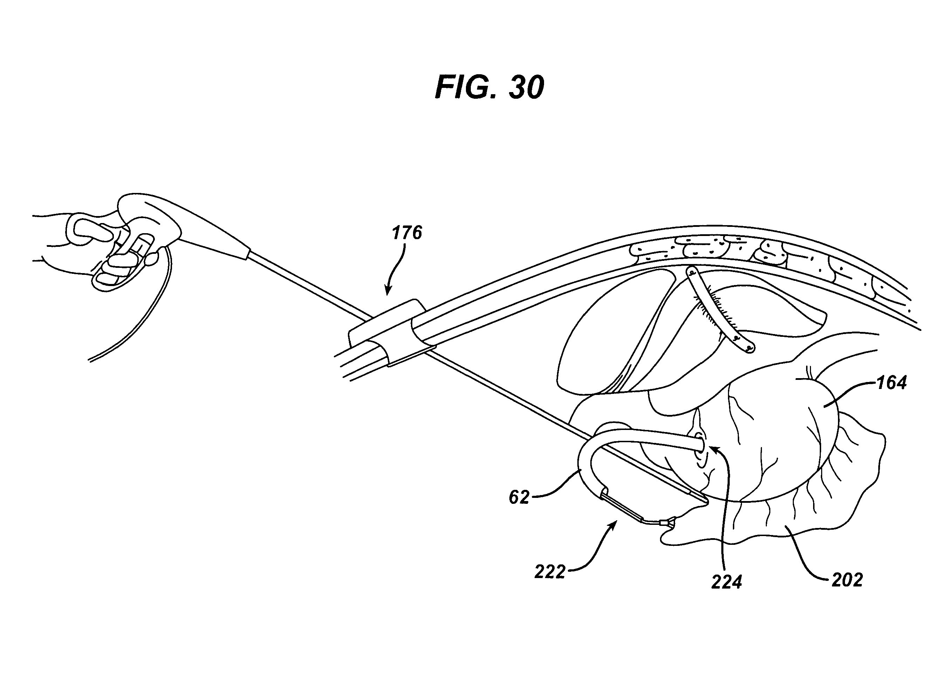

As illustrated in FIG. 30, which is similar to the alternate embodiment of FIG. 27 using the multiple port access device 176, a grasper 222 can be transorally advanced into the stomach 164, advanced through a digestive tract opening 224, and advanced into the abdominal cavity of the patient 10 to grab and tension the omentum 202. The digestive tract opening 224 can be formed in any way appreciated by a person skilled in the art. The digestive tract opening 224 can be formed at any location on the stomach 164, such as at the measured starting location for transection of the stomach 164, but it is preferably formed in a portion of the stomach 164 that will form part of the stomach sleeve following transection to help maintain constant positioning of any device(s) inserted through the digestive tract opening 224 before, during, and/or after transection. The digestive tract opening 224 is shown formed in the stomach wall, but the digestive tract opening 224 can be formed anywhere in the patient's digestive tract, e.g., in the stomach wall, in an intestine wall, etc. The digestive tract opening 224 can have any shape and size. If the digestive tract opening 224 is not included in a portion of the stomach fundus detached from a remainder of the stomach 164 during transection, the digestive tract opening 224 can be closed in any way appreciated by a person skilled in the art, e.g., using a surgical stapler inserted through an abdominally inserted multiple port access device.

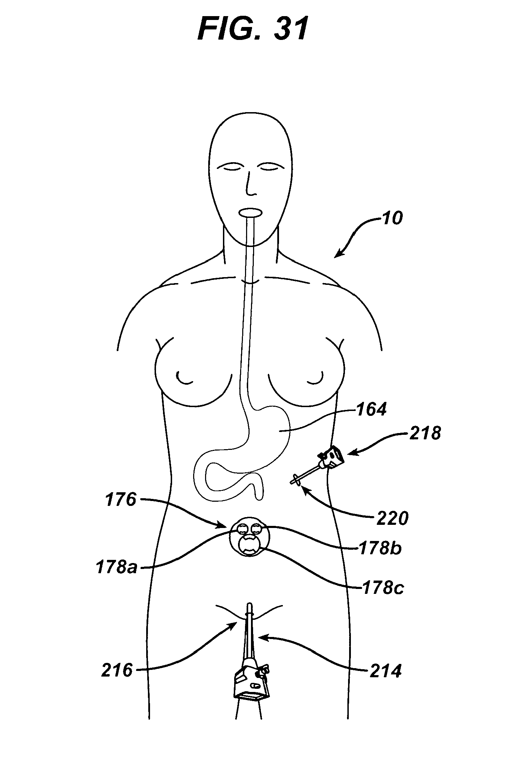

FIG. 31 shows an alternate embodiment, similar to the alternate embodiment of FIG. 21 using the abdominally-inserted multiple port access device 176 for dissecting tissue attached to the stomach 164. In this illustrated embodiment, the surgeon can use a scoping device, e.g., the laparoscope 181 of FIG. 21, advanced through a first one of the ports 178a, 178b, 178c in the multiple port access device 176 to visualize the surgical site, a dissector advanced through a second one of the ports 178a, 178b, 178c to dissect the tissue attached to the stomach 164, and a grasper advanced through a third one of the ports 178a, 178b, 178c to tension the tissue being dissected. Alternatively or in addition, a grasper advanced through a trocar 214 inserted through a percutaneous vaginal access hole 216 and/or a grasper advanced through a trocar 218 inserted through a percutaneous abdominal access hole 220 can be used to tension the tissue being dissected. A grasper inserted through at least the percutaneous abdominal access hole 220 can allow tissue to be tensioned in the patient 10 at a transverse angle relative to a surgical instrument, e.g., a cutting instrument, inserted into to the patient 10 through the multiple port access device 176 at the umbilicus.

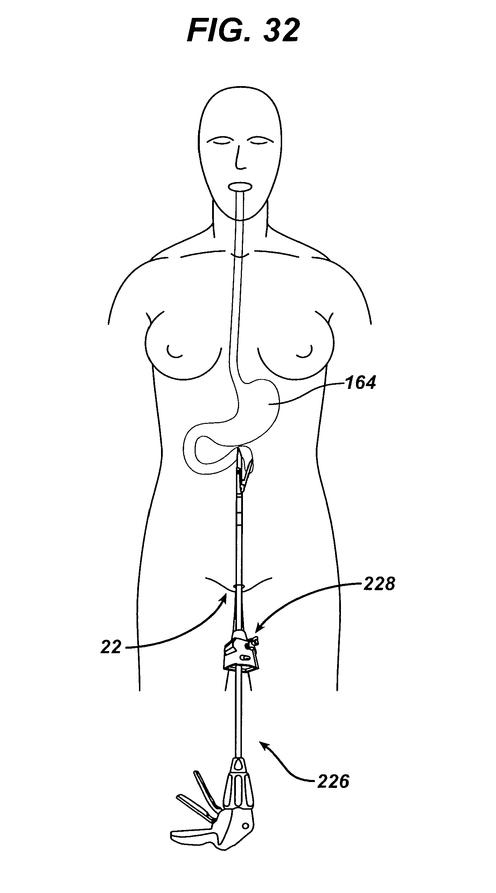

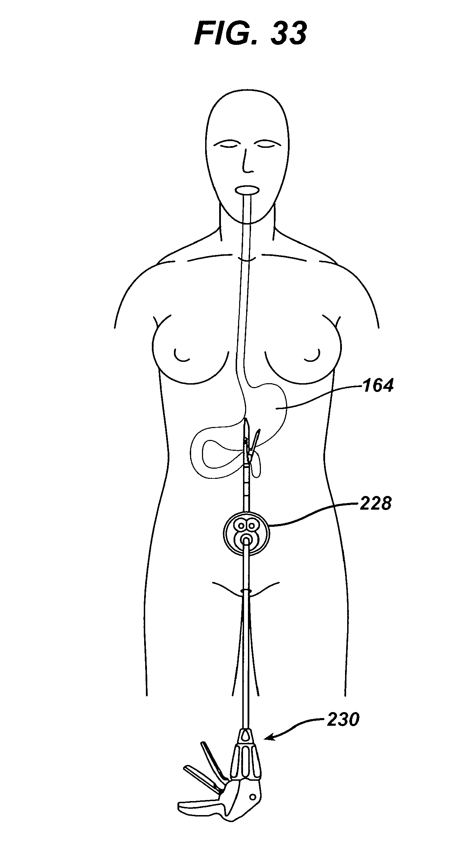

Once tissue attached to the stomach 164 is dissected from the stomach 164 as desired, the stomach 164 can be transected. As will be appreciated by a person skilled in the art, the stomach 164 can be transected using any one or more transecting devices. A person skilled in the art will also appreciate that the term "transector," "transecting device," or "transecting surgical instrument" as used herein is intended to encompass surgical devices that alone or in combination can cut and secure tissue, e.g., a surgical stapler configured to cut and staple tissue. Non-limiting embodiments of surgical staplers can be found in U.S. Patent No. 5,285,945 issued February 14, 1995 and entitled "Surgical Anastomosis Stapling Instrument," U.S. Patent No. 6,905,057 issued June 14, 2005 and entitled "Surgical Stapling Instrument Incorporating A Firing Mechanism Having A Linked Rack Transmission," U.S. Patent No. 7,111,769 issued September 26, 2006 and entitled "Surgical Instrument Incorporating An Articulation Mechanism Having Rotation About The Longitudinal Axis," U.S. Patent No. 6,786,382 issued September 7, 2004 and entitled "Surgical Stapling Instrument Incorporating An Articulation Joint For A Firing Bar Track," U.S. Patent No. 6,981,628 issued January 3, 2006 and entitled "Surgical Instrument With A Lateral-Moving Articulation Control," U.S. Patent No. 7,055,731 issued June 6, 2006 and entitled "Surgical Stapling Instrument Incorporating A Tapered Firing Bar For Increased Flexibility Around The Articulation Joint," U.S. Patent No. 6,964,363 issued November 15, 2005 and entitled "Surgical Stapling Instrument Having Articulation Joint Support Plates For Supporting A Firing Bar," U.S. Patent No. 6,959,852 issued November 1, 2005 and entitled "Surgical Stapling Instrument With Multistroke Firing Incorporating An Anti-Backup Mechanism," U.S. Patent Publication No. 2005/0070925 filed September 29, 2003 and entitled "Surgical Stapling Instrument Having Multistroke Firing With Opening Lockout," U.S. Patent No. 7,000,819 issued February 21, 2006 entitled "Surgical Stapling Instrument Having Multistroke Firing Incorporating A Traction-Biased Ratcheting Mechanism," and U.S. Patent No. 7,364,061 issued April 29, 2008 and entitled "Surgical Stapling Instrument Incorporating A Multistroke Firing Position Indicator And Retraction Mechanism," which are hereby incorporated by reference in their entireties. The transector can have any size and shape, but in an exemplary embodiment if the transector is vaginally advanced into the patient 10, the transector preferably has a relatively long longitudinal length, e.g., at least about 4 feet, and has at least one flexible joint. Non-limiting embodiments of a transector having at least one flexible joint can be found in previously mentioned U.S. Patent Application No. [ ] filed on even date herewith and entitled "Methods And Devices For Performing Gastroplasties Using A Multiple Port Access Device," [Atty. Docket No. 100873-319 (END6489USNP)]. In an exemplary embodiment, the transector is a linear surgical stapler configured to apply one or more rows of staples and to cut the stapled tissue. A person skilled in the art will also appreciate that the transector can be inserted into the patient 10 through any opening, e.g., through an abdominal access hole, a vaginal access hole, a natural orifice, etc., with or without a trocar or multiple port access device positioned therein. Further, at least one grasper inserted through any opening(s) in the patient 10 can be used to tension the stomach 164 while it is being transected and/or to hold a sizer in a desired location along the stomach's lesser curvature. In one embodiment illustrated in FIG. 32, the stomach 164 can be transected using a transecting device 226 advanced through a trocar 228 inserted in the vaginal access hole 22. In another embodiment illustrated in FIG. 33, the surgeon can transect the stomach 164 using a transecting device 230 advanced through a multiple port access device 228 inserted through the patient's umbilicus. The transection can be visualized using at least one scoping device inserted through any opening, as discussed herein. For non-limiting example only, the surgeon can visualize above and/or underneath the stomach 164 using, e.g., the laparoscope 20 inserted through the trocar 16 in the abdominal access hole 12, to determine if a desired path of transection is clear or readily cleared of tissue and/or other debris. For another non-limiting example, the surgeon can use one scoping device for visualization before the transection, e.g., the laparoscope 20 inserted through the trocar 16 in the abdominal access hole 12, and another scoping device during and after the transection, e.g., the vaginally introduced endoscope 62. The surgeon can also optionally tension the stomach during transaction. For example, a suture can be passed through a percutaneous opening, e.g., through a trocar or other port, and the suture can be inserted through the fundus of the stomach and back out the stomach and out the percutaneous port. The free ends of the suture can thus be tensioned to lift and stretch the stomach, thereby facilitating transaction. The surgeon can also place one or more draining devices in the stomach fundus following the transection, e.g., along a greater curvature of the stomach sleeve formed by the transection. If used, the sizer can be removed from the stomach 164 at any time during the surgical procedure, but in an exemplary embodiment the surgeon removes the sizer from the patient 10 by retracting it through the patient's mouth after the stomach 164 has been transected and inspected via scoping device visualization for any uncorrected and potentially dangerous irregularities, e.g., improperly bent staples, improperly placed staples, untied sutures, etc.

The surgeon can optionally secure the transected stomach, e.g., along the stapled or otherwise secured cut edge of the fundus, using any one or more supplemental securing elements in any combination to help better secure the transection and/or reduce bleeding. The supplemental securing elements are preferably biocompatible and can optionally be bioabsorbable such that the supplemental securing elements can dissolve in the patient 10 over time as the transection heals. Non-limiting embodiments of supplemental securing elements include sutures, glues such fibron glues, staples, pledgets, etc. The supplemental securing element(s) can be applied following the transection and/or the transector can be configured to apply one or more supplemental securing elements when it transects the stomach 164. Non-limiting embodiments of a surgical stapler than can apply staples with bioabsorbable pledgets can be found in previously filed U.S. Patent Application No. [Atty. Docket No. END5966], which is hereby incorporated by reference in its entirety.

As mentioned above, any portion of the stomach 164 can be transected. In one embodiment, the stomach 164 is fully transected to separate the stomach 164 and remove a portion of the fundus, leaving another portion of the fundus, the stomach sleeve that was sized by a sizer, to keep the patient's esophagus and pyloric valve in fluid communication. The surgeon can transect the stomach 164 in any way appreciated by a person skilled in the art, but in an exemplary embodiment, the surgeon uses the dissector to cut and secure the stomach 164 beginning at the starting location previously marked by the surgeon a distance from the pyloric valve. The surgeon can proceed to cut and secure the stomach 164 using the sizer as a guide from the starting location until an angle of HIS of the stomach 164 is breached, thereby forming a stomach sleeve. The detached, non-sleeve portion of the fundus can be removed from the patient 10 in any way, at any time following its detachment from the stomach sleeve, and through any opening in the patient 10, e.g., through an abdominally inserted multiple port access device, through a vaginal otomy, etc. For non-limiting example, the surgeon can use in combination an abdominally (or otherwise) inserted grasper and a vaginally (or otherwise) introduced specimen removal device, e.g., an Endopouch Retriever^{™} specimen removal bag available from Ethicon Endo-Surgery, Inc. of Cincinnati, Ohio, to guide the fundus into the specimen removal device and remove the detached fundus portion disposed in the specimen removal device from the patient 10.

In another embodiment, the stomach 164 is partially transected along a portion of the stomach 164 less than an entire length of the stomach 164, e.g., in a gastroplasty such as a Magenstrasse and Mill procedure. Such a partial transection can separate the fundus from an area of the stomach 164 substantially near the patient's esophagus and allow the fundus to retain fluid communication with the patient's pyloric valve. The surgeon can transect the stomach 164 by forming an opening through the stomach at the starting location, for example using a circular stapler. A transector can then be inserted through the stapled and cut opening to cut and secure the stomach, using the sizer as a guide, between the angle of HIS and a desired endpoint.

At the conclusion of a gastrectomy, any access holes formed in a patient can be closed in any way and in any order as will be appreciated by a person skilled in the art, such as by suturing the openings.

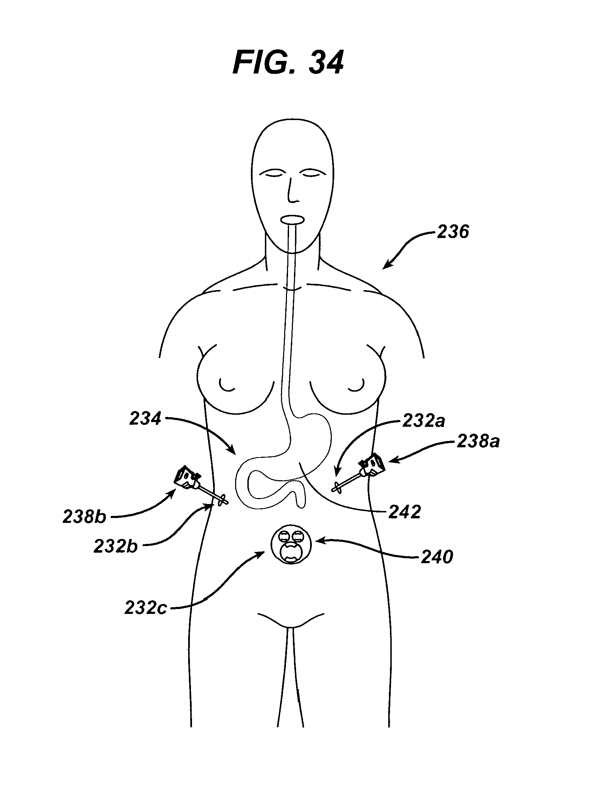

In some embodiments, for example with male patients, a gastrectomy procedure will not include forming a vaginal access hole. In these gastrectomies, one abdominal access hole can be formed in a patient that can accommodate a plurality of surgical instruments inserted therethrough, e.g., using a multiple port access device, or at least two abdominal access holes can be formed in a patient. FIG. 34 shows an exemplary embodiment of abdominal access holes arranged for such a gastrectomy. First, second, and third access holes 232a, 232b, 232c can be formed as percutaneous openings or otomies in any way through an abdominal wall 234 of a patient 236 to provide access to the patient's abdominal cavity, although any number of abdominal access holes can be formed in the patient 236. As illustrated, the first and second abdominal access holes 232a, 232b include percutaneous openings substantially laterally aligned on opposed sides of the patient's abdomen and having first and second trocars 238a, 238b respectively inserted therethrough, and the third abdominal access hole 232c includes an otomy located at the patient's umbilicus non-laterally aligned with and between the first and second abdominal access holes 232a, 232b and having a multiple port access device 240 inserted therethrough. The gastrectomy can be performed in any way discussed above, but in an exemplary embodiment, surgical instruments insertable through a vaginal opening can instead be inserted through one of the first and second abdominal access holes 232a, 232b. For non-limiting example, first and second graspers can be respectively inserted into the patient 236 through the first and second trocars 238a, 238b to allow for the graspers to approach a stomach 242 of the patient at different angles, while a scoping device, a transecting device, and a retracting device can be simultaneously and/or sequentially inserted into the patient 236 through the multiple port access device 240 to allow for instruments to approach the stomach 242 at angles different than those for instruments inserted through the first and/or second trocars 232a, 232b.

The patient 10 can optionally be provided with a drug and/or device that suppresses appetite that can work in conjunction with the stomach sleeve to help the patient 10 lose weight. Such a drug or device can be provided to the patient 10 at the end of the gastrectomy and/or in a subsequent surgical procedure. A non-limiting embodiment of an implantable appetite suppressant device is available from Duocore, Inc. of Ramat-Hasharon, Israel.

A gastrectomy procedure described herein can optionally be combined with one or more other surgical procedures. For non-limiting example, the gastrectomy can be combined with a transoral minimally invasive surgical procedure, non-limiting examples of which, e.g., creating a gastroenteroanastomosis or enteroenteroanastomosis, can be found in U.S. Patent Application No. 2006/0271075 filed May 18, 2006 and entitled "Double Loop Gastric Bypass Procedure," which is hereby incorporated by reference in its entirety. As another non-limiting example, the gastrectomy can be performed as a first stage of a two stage surgical procedure where a second stage, e.g., a duodenal switch, a Roux-en-Y procedure, etc., can be performed immediately after the gastrectomy or in a subsequent surgical procedure.

A person skilled in the art will appreciate that the present invention has application in conventional endoscopic and open surgical instrumentation as well application in robotic-assisted surgery.

The devices disclosed herein can also be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A surgical method, comprising:
forming a first access hole in an abdominal wall of a patient;
forming a second access hole through a digestive tract of the patient and into an abdominal cavity of the patient;
tensioning a tissue attached to a stomach of the patient using a first surgical instrument inserted through the second access hole;
detaching the tissue from the stomach using a second surgical instrument inserted through the first access hole; and
transecting a portion of the stomach using a surgical stapler inserted through the first access hole to form a stomach sleeve.

2. The method of claim 1, further comprising removing the transected portion of the stomach through the first access hole.

3. The method of claim 1, further comprising advancing a scoping device with a viewing element located thereon into the patient through the second access hole.

4. The method of claim 1, further comprising transorally introducing a sizing device into the stomach and using the sizing device to size the portion of the stomach to be transected.

5. The method of claim 1, wherein forming a first access hole comprises positioning a housing having a plurality of sealing ports in the abdominal wall.

6. The method of claim 1, wherein transecting a portion of the stomach comprises separating a fundus of the stomach from an area of the stomach substantially near an esophagus of the patient, wherein the fundus retains fluid communication with a pyloric valve of the patient.

7. The method of claim 1, wherein the second access hole is formed in the stomach sleeve.

8. The method of claim 1, further comprising retracting a liver of the patient using a device inserted through one of the first and second access holes.

9. A surgical method, comprising:
forming a first access hole in an abdominal wall of a patient;
forming a second access hole through a digestive tract of the patient and into an abdominal cavity of the patient;
inserting a scoping device through the second access hole;
tensioning a tissue attached to a stomach of the patient using a first surgical instrument inserted through the first access hole;
detaching the tissue from the stomach using a second surgical instrument inserted through the first access hole; and
transecting a portion of the stomach using a surgical stapler inserted through the first access hole.

10. The method of claim 9, further comprising removing the transected portion of the stomach from the patient through the first access hole.

11. The method of claim 9, wherein forming a first access hole comprises positioning a housing having a plurality of sealing ports in the abdominal wall.

12. The method of claim 9, further comprising transorally introducing a sizing device into the stomach and using the sizing device to size the portion of the stomach to be transected.

13. The method of claim 9, wherein transecting a portion of the stomach comprises separating a fundus of the stomach from an area of the stomach substantially near an esophagus of the patient, wherein the fundus retains fluid communication with a pyloric valve of the patient.
